(19) Europäisches Patentamt
European Patent Office
Office européen des brevets

(11) Numéro de publication: **0 090 100**
**B1**

(12) # FASCICULE DE BREVET EUROPEEN

(45) Date de publication du fascicule du brevet: **17.09.86**

(51) Int. Cl.⁴: **C 08 F 8/32,** C 08 J 7/14, C 08 B 37/00, A 61 F 2/00

(21) Numéro de dépôt: **82201541.8**

(22) Date de dépôt: **11.07.80**

(60) Numéro de publication de la demande initiale en application de l'article 76 CBE: **0023854**

(54) **Polymères insolubles ayant des propriétés anticoagulantes, objets les contenant et procédés de fabrication.**

(30) Priorité: **20.07.79 FR 7918780**

(43) Date de publication de la demande: **05.10.83 Bulletin 83/40**

(45) Mention de la délivrance du brevet: **17.09.86 Bulletin 86/38**

(84) Etats contractants désignés: **AT BE CH DE FR GB IT LI LU NL SE**

(56) Documents cités:
**DE - A - 2 728 781**
**FR - A - 2 066 460**
**FR - A - 2 078 435**
**US - A - 2 808 405**
**US - A - 3 301 848**

**CHEMICAL ABSTRACTS, vol. 77, no. 18, 30 oktobre 1972, no. 115177v, Columbus Ohio USA; R. MOROSCIKA: "Dissymmetric ion-exchange resins derived from alpha-amino acids and chlorosulfonated styrene-divinylbezene copolymers"**

(73) Titulaire: **Fougnot, Christine, 85, Rue Marcel Grandcoing, F-93430 Villetaneuse (FR)**
Titulaire: **Jozefonvicz, Jacqueline, née Dorgebray, 65, 2ème Avenue, F-60260 Lamorlaye (FR)**
Titulaire: **Jozefonvicz, Marcel, 65, 2ème Avenue, F-60260 Lamorlaye (FR)**

(72) Inventeur: **Fougnot, Christine, 85 rue Marcel Grandcoing, F-93430 Villetaneuse (FR)**
Inventeur: **Jozefonvicz, née Dorgebray, Jacqueline, 65 2ème Avenue, F-60260 Lamorlaye (FR)**
Inventeur: **Jozefonvicz, Marcel, 65 2ème Avenue, F-60260 Lamorlaye (FR)**
Inventeur: **Mauzac, née Massat, Monique, 46 Rue Peyronnet, F-92200 Neuilly-sur-Seine (FR)**

(74) Mandataire: **Ores, Irène et al, CABINET ORES 6, Avenue de Messine, F-75008 Paris (FR)**

**EP 0 090 100 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention est relative à des polymères substitués par des groupes leur conférant des propriétés anticoagulantes et à leur procédé de préparation, aux objets constitués par et/ou comprenant lesdits polymères substitués, à leurs procédés de fabrication et à l'application desdits objets en chirurgie et en médecine, et aux compositions pharmaceutique contenant lesdits polymères substitués.

L'héparine, agent anticoagulant naturel du sang, constitue un médicament de choix dans les affections thromboemboliques. Aussi, en vue de pallier les inconvénients de l'héparine, depuis un certain nombre d'années déjà, on a essayé de suppléer cette précieuse matière par divers produits synthétiques ou semi-synthétiques.

Ainsi, on a préconisé: – le sulfate de chondroïtine, (qui est également un polyholoside hétérogène), – le chitosane (produit de dédoublement de la chitine) préparé par la N-désacétylation totale de la chitine [HORTON et JUST «Carbohydrate Research» 29 (1973) 173–179], – le chitosane sulfaté [WOLFROM et SHENHAN, JACS (1959) 81, 1764–1766], – le sulfate d'héparane [JORPES et GARDELL «J. Biol. Chem.» 176–267 (1948)] et d'autres polysaccharides formés par la polymérisation d'une unité disaccharidique constituée par une osamine (principalement la D-glucosamine) portant un certain nombre de groupements sulfate et/ou sulfonate et par un acide uronique (généralement l'acide D-glucuronique et l'acide L-iduronique). Outre le fait que l'action héparinoïde («heparin-like») de ces divers produits est moins prononcée que celle de l'héparine, leur origine naturelle est la cause (comme c'est le cas pour l'héparine) de leur variabilité et de leur hétérogénéité d'une préparation à l'autre.

Une autre série d'héparinoïdes est constituée par des produits dérivés de l'amylose (lequel, comme l'héparine a des liaisons $\alpha$ (1 → 4), qui ont été étudiés, entre autres, par WOLFROM et WANG (Carbohydrate Research 18 (1971) 23–27) concernant l'amylose aminé et sulfaté et par HORTON et JUST (Carbohydrate Research 30 (1973) 349–357) concernant la conversion de l'amylose en 1 → 4) 2-amino-2-déoxy-$\alpha$D-glucopyranuronane. Les produits ainsi obtenus ont une activité héparinique faible ou pratiquement nulle (amylose aminé et sulfaté).

Certains autres polysaccharides ont été signalés pour leurs propriétés plus ou moins anticoagulantes: – le sulfate de dextrane [V. POTUZNIK J. Hyg. Epid. Microbiol. Immunol. 16, 293 (1972)]; – le polyester sulfurique de pentosane [produit vendu sous le nom de «HEMOCLAR» par les Laboratoires CLIN-MIDY]; ou les dérivés de l'acide alginique [O. LARM et collab. Carbohydr. Res. 73, 332 (1979)]. Malheureusement, aucun de ces produits ne peut être valablement comparé à l'héparine.

On a également proposé (FR-A-2 280 387) des dérivés sulfonés de polypeptides, à savoir des copolymères de la lysine et du tryptophane. Si ces produits provoquent effectivement un allongement très net du temps de coagulation et ont une action du même type que celle de l'héparine, ils ne peuvent pas être utilisés sur une grande échelle, car les réactions secondaires qu'ils provoquent dans l'organisme humain sont extrêmement gênantes.

En 1975, Harry P. GREGOR [Polym. Sci. Technol. U.S.A. (1975), 7, 51–56] a préconisé l'utilisation, en tant que matériaux «heparin-like», de polymères et de copolymères sulfonés (en particulier des acides polystyrènesulfonique et polyéthylènesulfonique). T. BEUGELING et Collab. [J. BIOMED. MATER. RES. (1974) Vol. 8, 375–379 et BIOCOMPAT. IMPLANT. MATER, 187–192 (1976] ont préconisé l'utilisation de polymères synthétisés à partir de polyisoprènes, dont le motif est représenté comme suit:

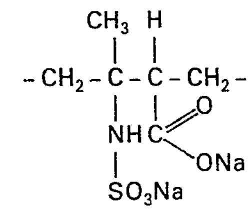

(I)

qui sont des dérivés contenant des groupes aminosulfonates et carboxylates.

Ces Auteurs ont obtenu essentiellement des dérivés solubles dont l'activité anticoagulante ne représente cependant que 12 à 15% de celle de l'héparine prise comme référence.

D'autres tentatives intéressantes ont été effectuées par greffage de la molécule d'héparine elle-même sur la surface des polymères [par exemple à l'aide de l'agent de couplage constitué par le chlorure de tridodécylméthylammonium: LEININGER et Collab. dans Trans. Amer. Soc. Artif. Int. Organs 18 312 (1972)] ou par liaison covalente: [HOFFMAN et Collab. dans Trans. Amer. Soc. Artif. Int. Organs 18 10 (1972)]. Malheureusement, outre le fait que l'on ne parvient à fixer qu'une quantité relativement faible d'héparine, ces polymères «héparinisés» ne sont pas stables: la quantité fixée a tendance à s'inactiver avec le temps.

D'une manière générale, l'intérêt qu'il y aurait à pouvoir disposer d'un produit insoluble à action anticoagulante non seulement stable, reproductible et homogène d'un lot à l'autre, est à l'origine des nombreux travaux de recherche de nouveaux anticoagulants.

C'est ainsi qu'on a obtenu des produits très actifs par greffage chimique ou radiochimique de monomères vinyliques sur l'héparine [cf. les travaux de C. BAQUEY et Collab. dans Ann. Phys. Biol. Med. 9 (2) 131–138 (1975) et de D. LABARRE, Thèse de Doctorat d'Etat Paris (1977)]. Les polymères insolubles ainsi obtenus sont inutilisables dans la pratique, car en plus de l'inactivation qu'elle subit avec le temps, l'héparine greffée migre à l'intérieur de la molécule au cours de la mise en œuvre de ces produits, et se trouve totalement masquée.

Les Demandeurs ont formulés l'hypothèse selon laquelle les propriétés anticoagulantes de l'héparine ne sont pas liées à sa structure secondaire ou tertiaire, mais plus précisément à la nature des différents groupes portés par la chaîne polysaccharide et à la combinaison des effets de ces groupes, qui aurait un résultat coopératif multiplicateur de l'activité de chacun d'entre eux vis-à-vis de la thrombine et de l'antithrombine III.

On considère que l'héparine, généralement représentée par la formule II ci-après:

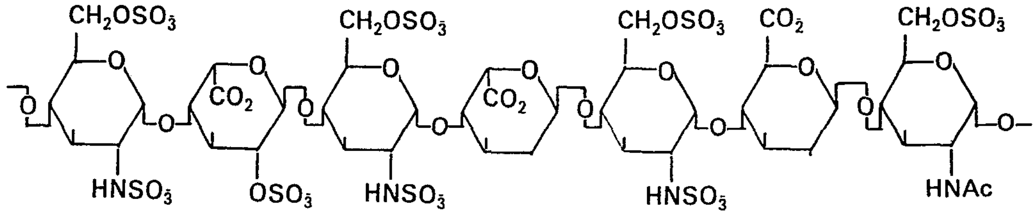

(II)

possède également des motifs non O-sulfatés ou 2-O sulfatés du résidu uronique

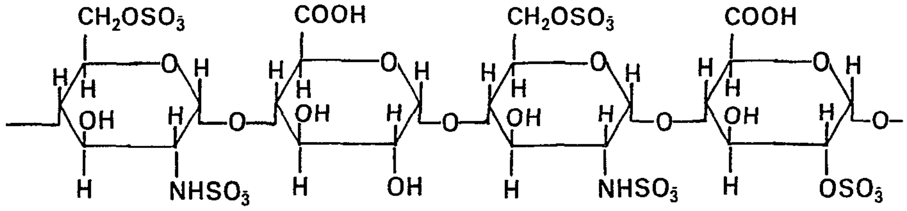

(III)

Par ailleurs, on a préparé des résines échangeuses d'ions utiles pour la chromatographie liquide des mélanges racémiques, lesquelles résines sont essentiellement constituées par des polystyrènes sur lesquels ont été fixés les acides aminés suivants: l'alanine, la valine, la norvaline, la phénylalanine, la leucine, l'isoleucine, la tyrosine, la sérine, la thréonine, l'acide aspartique, la proline, l'hydroxyproline et l'acide glutamique [cf. en particulier les travaux effectués par VESA et Collab., Zh. Obsch. Khim. SSSR 42 (12) 2780 (1972) et Tr. Akad. Nauk. Lit. SSR. Ser. B 2-69, 93 (1972) et par PETIT ET JOZEFONVICZ (Jour. Of. Applied Polymer Sci. Vol. 21 2589-2596 (1977)].

Partant de leur hypothèse relative à l'importance de la présence simultanée des groupes qui figurent dans les formules II et III ci-dessus, sur un support macromoléculaire, les Demandeurs ont pu mettre en évidence que de telles résines échangeuses d'ions présentent des applications en tant qu'agents doués d'une action anticoagulante.

Mieux encore, par leur compréhension de ce phénomène et en poussant plus loin leurs études et leurs investigations, les Demandeurs ont pu établir une formule plus générale convenant à tous les polymères réticulés et comportant dans leur chaîne des groupes substituables. Ils ont pu aboutir ainsi à des produits totalement insolubles, présentant des propriétés anticoagulantes.

La présente invention a pour objet des produits doués de propriétés anticoagulantes, caractérisés en ce qu'ils sont constitués par des polymères, homopolymères ou copolymères réticulés et insolubles choisis parmi le polystyrène et les polysaccharides, comportant dans leur chaîne des groupes substituables sur lesquels sont fixés de façon statistique des groupes X et Y et/ou V, où

X désigne le groupe $-SO_3R_1$ ou $-R_3-SO_3R_1$

$R_1$ étant un atome d'hydrogène ou d'un métal physiologiquement compatible,

$R_3$ étant un groupe $-CH_2-CO-NH-R_4-$ dans lequel $R_4$ représente un radical alkylène, arylène ou alkylarylène substitué ou non

ou $-CH_2$ 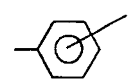 substitué ou non,

Y désigne le groupe $-SO_2-R_2$ ou $-R_3-SO_2-R_2$,

$R_2$ étant le reste d'un acide aminé quelconque dans le cas des polysaccharides et choisi dans le cas du polystyrène dans le groupe qui comprend la méthionine, la cystéine, l'acide cystéique, la proline, l'hydroxyproline, la sérine, la tyrosine, la benzyloxycarbonyl-lysine, la tertiobutyloxycarbonyl-lysine, l'acide ε-aminocaproïque, la β-alanine, l'acide γ-amino-n-butyrique, l'acide δ-amino-n-valérique, substitués ou non, lequel acide aminé est lié au pont $-SO_2-$ par sa fonction amine et

V désigne le groupe $-CH_2-CO-NH-CHR-COOH$,

R étant la chaîne latérale d'un acide aminé,

étant bien entendu que:

a) si X est $-SO_3R_1$, il est obligatoirement accompagné de Y et/ou de V, et

b) V est toujours accompagné de X et/ou de Y.

Conformément à l'invention, les acides aminés sont choisis parmi ceux qui comportent au moins une fonction carboxylique libre et s'ils possèdent plusieurs fonctions amine, toutes sauf une doivent être bloquées par un groupe électroattracteur physiologiquement acceptable.

Suivant une modalité particulière de ce mode de réalisation, le groupe électroattracteur est avantageusement constitué par le groupe benzyloxycarbonyle ou tertiobutyloxycarbonyle.

Suivant un mode de réalisation avantageux de l'objet de la présente invention, les polymères

portant des groupes X, et/ou Y et/ou V fixés, sont des polymères réticulés.

Les produits ainsi obtenus, qui sont insolubles dans l'eau et dans les fluides biologiques, permettent de façonner divers objets tels que des prothèses cardiovasculaires, des cathéters et des fils de suture en matériau anticoagulant.

Selon un mode de réalisation avantageux de l'objet de la présente invention, les polymères réticulés, comportant dans leur chaîne des groupes substituables sur lesquels sont fixés les groupes S et/ou Y et/ou V, sont des polystyrènes.

Selon un autre mode de réalisation avantageux de l'objet de l'invention, les polymères réticulés, comportant dans leur chaîne des groupes substituables sur lesquels sont fixés les groupes X et/ou Y et/ou V, sont des polysaccharides.

Suivant une modalité particulière de ce mode de réalisation, les polymères sont constitués par des dextranes. La biocompatibilité des dextranes avec le sang est connue depuis bien longtemps. A. GRONWALL et Collab. [Acta. Physiol. Scand. 7 97 (1944)] ont préconisé leur utilisation comme substitut du plasma sanguin et W. APPEL et Collab. [Angew. Chem. Intern. Ed. 7, 702 (1968)] ont étudié leur dégradation enzymatique dans le plasma et les tissus sous l'action de la dextranase.

La présente invention a également pour objet un procédé de préparation des produits doués d'une action anticoagulante conformes à la présente invention, lequel procédé, dans le cas où l'on désire obtenir les produits dans lesquels:

$X = -SO_3R_1$
$Y = -SO_2-R_2$ et
V est nul,

($R_1$ et $R_2$ ayant la même signification que ci-dessus), se caractérise en ce qu'au cours d'une première étape, on prépare un polymère chlorosulfoné POL-$SO_2Cl$ par réaction de l'acide chlorosulfonique sur le polymère dans un solvant approprié, et en ce qu'au cours d'une deuxième étape on transforme les groupes $-SO_2Cl$ en groupes $-SO_3Na$ et en groupes $SO_2AA$ (où AA représente un acide aminé) par réaction avec une quantité appropriée d'un acide aminé en milieu basique.

Suivant une modalité particulière de ce mode de réalisation, dans le cas où on désire obtenir des polymères substitués insolubles à action anticoagulante, la sulfonation du polymère réticulé a lieu dans un mélange contenant du dichlorométhane et du nitrométhane, et la réaction avec l'acide aminé a lieu dans un milieu contenant le mélange eau-dioxane.

Suivant une modalité particulière de ce mode de réalisation et afin d'éliminer le plus complètement possible toute impureté susceptible d'interagir avec les facteurs de la coagulation, on procède dans le cas de polymères insolubles, après la fixation des acides aminés, à un lavage abondant à l'eau suivi de lavages par une solution de NaCl (1,5 M), puis de lavages au citrate de Na (1 M), d'un équilibrage à pH 7,30 environ par plusieurs lavages avec du tampon de MICHAELIS, un nouveau lavage à l'eau et enfin un séchage.

Conformément à l'invention, dans le cas où l'on désire obtenir les produits dans lesquels

$X = -R_3-SO_3R_1$
$V = -CH_2-CO-NH-CHR-COOH$ et
Y est nul,

($R_1$ et $R_3$ ayant la même signification que ci-dessus), le procédé se caractérise en ce que l'on prépare au cours d'une première étape les dérivés carboxyméthylés des polymères, en ce que l'on fixe au cours de la deuxième étape les amines appropriées ou le chlorure de benzyle, en ce que l'on fixe les acides aminés au cours d'une troisième étape et en ce que l'on procède à la sulfonation au cours d'une quatrième étape.

Suivant une modalité particulière de ce mode de réalisation, le couplage des amines et/ou des acides aminés sur des dérivés carboxyméthylés des polymères, s'effectue à l'aide de N-éthoxycarbonyl-2-éthoxy 1,2-dihydroquinoléine (EEDQ) ou autre agent de couplage analogue.

Suivant une autre modalité particulière de ce mode de réalisation, l'étape de sulfonation est répétée plusieurs fois si l'on désire augmenter le taux de groupements sulfonates.

Conformément à l'invention, dans le cas où l'on désire obtenir les produits dans lesquels:

$X = -R_3-SO_3R_1$
$V = -CH_2-CO-NH-CHR-COOH$ et
$Y = -R_3-SO_2-R_1$

R, $R_1$, $R_2$ et $R_3$ ayant la même signification que ci-dessus, le procédé se caractérise en ce qu'au cours d'une première étape on procède à la carboxyméthylation, en ce qu'au cours d'une deuxième étape on fixe les amines appropriées ou le chlorure de benzyle, en ce qu'au cours d'une troisième étape on procède à la chlorosulfonation, en ce qu'au cours de la quatrième étape on fixe les acides aminés.

La présente invention a également pour objet des articles à usage médical et chirurgical tels que tubes, prothèses cardiovasculaires, cathéters, fils et films en polymères comportant des groupes substituables, rendus anticoagulants par fixation sur leur surface extérieure des groupes X, et/ou Y, et /ou V tels que définis précédemment. Autrement dit, il suffit tout d'abord de façonner un objet de forme et de dimensions voulus à partir d'un polymère comportant des groupes substituables, puis de fixer chimiquement (en mettant en œuvre, par exemple, le procédé décrit plus haut) les groupes X, et/ou Y, et/ou V en quantités voulues et aussi élevées qu'on le désire, pour obtenir un matériau solide à action anticoagulante, très stable dans le temps, ne subissant aucun relargage ni dégradation.

La présente invention a en outre pour objet des compositions pharmaceutiques constituées par, ou contenant les polymères non réticulés substitués par des groupes leur conférant une action

anticoagulante et solubles dans l'eau, lesdits polymères étant présents dans lesdites compositions pharmaceutiques à raison d'une dose thérapeutiquement active.

Outre les dispositions qui précèdent, l'invention comprend encore d'autres dispositions qui ressortiront de la description qui va suivre.

La présente invention vise plus particulièrement l'utilisation des polymères comportant des groupes X, et/ou Y, et/ou V tels que définis plus haut, fixés sur leur chaîne macromoléculaire, en tant que des objets fabriqués à partir de ces polymères, présentant des propriétés analogues à celle de l'héparine.

L'invention pourra être mieux comprise à l'aide du complément de description qui va suivre, qui se réfère à des exemples de préparation et de caractérisation des produits objets de la présente invention, à une étude de l'activité anticoagulante, et d'autres propriétés analogues à celles de l'héparine de ces produits, et à une évaluation de l'activité propre de chaque type de substituant.

Il doit être bien entendu, toutefois, que les différents exemples, caractéristiques et études qui seront decrits ci-après et représentés sur les dessins annexés, sont donnés uniquement à titre d'illustration de l'objet de l'invention, mais n'en constituent en aucune manière une limitation.

I - Préparation des matériaux et réactifs ayant le Polystyrène (PS) comme Polymère support

Les matériaux suivants ont été préparés:

a) produits à action anticoagulante conformes à l'invention

| N° de référence | Nom du produit |
|---|---|
| 1 | PS-hydroxyproline |
| 2 | PS-proline |
| 6 | PS-méthionine |
| 8 | PS-α ou ε-benzyloxycarbonyllysine |
| 9 | PS-ε-tertiobutyloxycarbonyllysine |
| 17 | PS-β alanine |
| 18 | PS-acide ε-aminocaproïque |

b) Produits d'étude et de comparaison

| N° de référence | Nom du produit |
|---|---|
| 3 | PS-alanine |
| 4 | PS-phénylalanine |
| 5 | PS-acide glutamique |
| 7 | PS-thréonine |
| 10 | PS-lysine (diamino-acide) |
| 11 | PS–$CH_2$-proline (l'acide aminé est fixé par l'intermédiaire du pont $-CH_2-$) |
| 12 | PS–$CH_2$-hydroxyproline (l'acide aminé est fixé par l'intermédiaire du pont $-CH_2-$) |
| 13 | PS–$CH_2$-alanine (l'acide aminé est fixé par l'intermédiaire du pont $-CH_2-$) |
| 14 | PS-butylamine (fixation d'une amine et non plus d'un acide aminé) |
| 15 | PS–$SO_3$ (résine sulfonée seulement) |
| 16 | PS-acide aspartique |

Le polystyrène de départ est un copolymère de styrène et de 2% de divinylbenzène, commercialisé par FLUKA. Il est utilisé sous forme de billes de 200 à 400 mesh de diamètre soit entre 0,037 et 0,074 mm de diamètre. Le produit commercial est lavé successivement avec une solution 1 M de NaOH, de l'eau, une solution 1 M de HCl et de l'eau. Il est séché sous vide à 60°C.

Les acides aminés utilisés sont des réactifs «FLUKA Puriss».

c) Préparation du PS–$SO_3$ (15)

On laisse gonfler une nuit, à la température ambiante, 25 g de polystyrène dans 200 ml de dichlorométhane. On ajoute alors un mélange de 160 ml de nitrométhane et de 140 ml d'acide chlorosulfonique. La suspension est agitée pendant 7 heures à 40°C. La résine brute est alors filtrée, lavée avec précaution avec du nitrométhane et de l'acétone. Elle est finalement séchée sous vide à 50°C.

Le taux de groupes chlorosulfonyle ($-SO_2Cl$) est déterminé de la façon suivante: 200 mg de polystyrène chlorosulfoné sont hydrolysés avec 50 ml d'une solution 1 M de NaOH pendant 24 heures au reflux. Après acidification, les ions $Cl^-$ sont titrés par une solution 0,1 M de $AgNO_3$ en utilisant une électrode indicatrice d'argent.

Le polystyrène sulfoné est hydrolysé quantitativement par la soude 2 M à la température ambiante. On filtre, on lave à l'eau et on sèche sous vide.

d) Préparation des produits 1, 2, 6, 8, 9, 17 et 18

86 mmoles de l'acide aminé sont dissoutes dans 130 ml du mélange 3:2 eau-dioxane par addition du minimum de soude 4 M. Le pH est mesuré et on ajoute alors 10 g du polystyrène chlorosulfoné tel qu'obtenu plus haut (43 meq $-SO_2Cl$). Le pH est ensuite maintenu à sa valeur initiale par addition de soude 2 M. La réaction est arrêtée quand le pH reste stable. Le polymère est alors filtré, abondamment lavé à l'eau, avec de la soude $10^{-2}$ M et de l'eau. Il est enfin séché sous vide.

e) Préparation d'une résine polystyrène contenant seulement des groupes $-SO_2$ aminoacide et pas de groupes sulfonate ($-SO_3R_1-$)

86 mmoles de l'ester méthyliques de l'acide aminé sont dissoutes dans 250 ml de dichlorométhane. On ajoute alors 10 g du polystyrène chlorosulfoné tel qu'obtenu plus haut. Le mélange est agité pendant 48 heures à 40°C. Le polymère est alors filtré, abondamment lavé à l'éthanol puis séché sous vide. L'hydrolyse de l'ester méthylique est effectuée dans la soude 2 M et conduit à la résine contenant exclusivement des groupes

sulfamide d'aminoacide dont les fonctions carboxyliques sont libres.

f) Préparation des polymères PS-$CH_2$-AA 11, 12, 13 (dans lesquels l'acide aminé est fixé au polystyrène par l'intermédiaire du pont $-CH_2-$)

1ère étape: Préparation du polystyrène chlorométhylé

On fait gonfler pendant 30 minutes, 25 g de polystyrène réticulé à 25°C dans 150 ml de chloroforme. On ajoute alors 100 ml d'éther monochlorodiméthylique et 10 ml de chlorure stannique. On laisse la réaction se poursuivre 1,5 heure sous agitation à la température ambiante. La résine est alors filtrée, lavée avec un mélange 3/1 de dioxane/eau, puis avec un mélange 3/1 de dioxane/acide chlorhydrique 3 N. Le polymère est ensuite lavé avec des mélanges eau/dioxane de plus en plus riches en dioxane, avec du dioxane, puis avec des mélanges dioxane/méthanol de plus en plus riches en méthanol, et enfin avec du méthanol. La résine est alors séchée sous vide à 50°C.

Le taux des groupes chlorométhyle ($-CH_2Cl$) est déterminé de la façon suivante: 200 mg de polystyrène chlorométhylé sont quaternisés dans 5 ml de butylamine pure à l'ébullition pendant 6 heures. Après acidifiaction du milieu par l'acide nitrique, les ions $Cl^-$ sont titrés par une solution 0,1 M de $AgNO_3$ en utilisant une électrode indicatrice d'argent.

2ème étape: Obtention du sel de sulfonium

20 g de polystyrène chlorométhylé (80 mmoles) sont mis en suspension dans un mélange de 30 ml de diméthylsulfure (400 mmoles), 100 ml d'eau et 120 ml d'isopropanol. Le mélange est agité pendant 48 heures à la température ambiante. Le rendement de la réaction est déterminé par titration potentiométrique des ions $Cl^-$ contenus dans une partie aliquote du milieu réactionnel. Ce rendement est d'environ 80%. Le sel de sulfonium n'est pas isolé.

3ème étape: Fixation de l'amino-acide

A la suspension précédente, on ajoute 64 mmoles de soude et 120 mmoles du sel de sodium de l'amino-acide dans 170 ml d'isopropanol et 200 ml d'eau [rapport AA/groupes $-CH_2S^+(CH_3)_2=2$]. Le mélange réactionnel est agité au reflux pendant 24 heures. La résine brute est alors filtrée et remise en suspension dans l'ammoniaque 4 M au reflux pendant 4 heures, pour éliminer le diméthylsulfure. Le polymère PS-$CH_2$-AA est alors filtré, lavé successivement avec de l'eau, de l'HCl 1 M, de l'eau et de la soude 1 M. Il est ensuite lavé plusieurs fois à l'eau jusqu'à ce que le filtrat soit neutre. Le polymère est alors séché sous vide.

Les caractéristiques des produits préparés selon d) et f) sont résumés dans le Tableau I qui va suivre.

Tableau I

| Amino-acide fixé | Rapport $AA/SO_2Cl$ | AA (meq/g) | $SO_3^-$ (meq/g) | Activite ($meq^{-1}$) |
|---|---|---|---|---|
| 1 | 1,6 | 2,23 | 0,93 | 91 |
| | 0,6 | 1,90 | 1,37 | 126 |
| | 0,4 | 1,37 | 2,16 | 179 |
| | 0,2 | 0,67 | 3,17 | 43 |
| 2 | 2 | 2,59 | 0,48 | 119 |
| | 1,5 | 2,08 | 1,14 | 91 |
| | 1 | 2,10 | 1,05 | 86 |
| | 0,6 | 1,63 | 1,83 | 78 |
| | 0,3 | 0,93 | 2,99 | 50 |
| 3 | 2 | 2,78 | 0,67 | 70 |
| | 1,5 | 2,58 | 0,90 | 106 |
| | 1 | 2,20 | 1,24 | 57 |
| | 0,3 | 0,81 | 3,08 | 48 |
| 4 | 2 | 2,21 | 0,79 | 59 |
| | 1,5 | 2,14 | 0,79 | 60 |
| | 1 | 2,03 | 1,15 | 50 |
| | 0,6 | 1,51 | 1,73 | 47 |
| | 0,3 | 0,90 | 2,92 | 42 |
| 5 | 2 | 1,33 | 2,02 | 214 |
| | 1,2 | 1,5 | 1,80 | 136 |
| | 1,6 | 1,0 | 2,32 | 89 |
| | 0,8 | 0,82 | 2,63 | 71 |
| | 0,7 | 0,31 | 3,43 | 84 |
| 6 | 2 | 2,34 | 0,43 | 119 |
| | 1,8 | 2,28 | 0,49 | 83 |
| | 1,3 | 1,89 | 0,94 | 100 |
| | 1 | 1,69 | 1,37 | 77 |

Tableau I (suite)

| Amino-acide fixé | Rapport AA/$SO_2Cl$ | AA (meq/g) | $SO_3^-$ (meq/g) | Activite (meq-¹) |
|---|---|---|---|---|
| | 0,8 | 1,45 | 1,90 | 62 |
| | 0,7 | 1,24 | 2,14 | 68 |
| 7 | 2 | 2,41 | 0,93 | 120 |
| | 1,6 | 2,38 | 1,01 | 127 |
| | 1,2 | 2,22 | 1,21 | 107 |
| | 0,8 | 2,02 | 1,53 | 95 |
| | 0,6 | 1,58 | 1,97 | 52 |
| | 0,3 | 0,79 | 2,86 | 55 |
| 8 | 1 | 1,41 | 1,20 | 75 |
| | 2 | 1,34 | 1,34 | 58 |
| | 0,6 | 1,11 | 1,87 | 65 |
| | 0,3 | 0,75 | 2,59 | 63 |
| 9 | 1 | 0,95 | 2,30 | 58 |
| | 0,6 | 0,88 | 2,53 | 52 |
| | 1 | 0,75 | 2,64 | 49 |
| | 2 | 1,03 | 2,22 | 99 |
| 10 | 1 | 1,56 | 1,94 | 36 |
| 11 | 2 | 1,2 | - | - |
| 12 | 2 | 1,0 | - | - |
| 13 | 1,4 | 0,8 | - | - |
| 14 | 1 | 3,14 | 0,88 | 11 |
| | 0,6 | 2,08 | 1,94 | 22 |
| | 0,5 | 1,77 | 2,21 | 50 |
| | 0,3 | 1,09 | 3,01 | 45 |

g) Conditionnement et granulométrie

Le polystyrène de départ est une résine commerciale dont les grains, sphériques, ont un diamètre moyen de 30 à 80 μm. La fixation des différentes fonctions modifie peu la taille des grains à l'état sec, mais augmente sensiblement leurs dimensions en suspension en milieu aqueux, puisque ces fonctions donnent au polystyrène ainsi modifié un caractère hydrophile marqué.

Une étude préliminaire ayant montré que l'activité anticoagulante est fonction de la surface spécifique des grains, celle-ci a été augmentée par broyage. Les échantillons broyés ont ensuite été mis en suspension dans du tampon de MICHAELIS et fractionnés selon la taille des grains, afin d'éliminer les particules d'un diamètre moyen inférieur à 2 μ environ qui restent en suspension quasi-colloïdale.

Ainsi, les billes de différents polymères brutes obtenues, sont tout d'abord lavées, équilibrées à pH 7,35 (tampon de MICHAELIS), puis séchées. Les billes de résine sont ensuite broyées (par déplacements très rapides de billes d'agate dans un moule d'agate), afin de réduire la taille des particules étudiées. Pour éliminer les très fines particules, le produit brut est lavé plusieurs fois par mise en suspension dans du tampon de MICHAELIS, suivie d'une décantation et de l'élimination du surnageant. La distribution granulométrique des échantillons obtenus est déterminée par microscopie quantitative («Technic Analysis System»). Ce fractionnement en fonction de la taille des particules, rend possible l'étude de l'acitivité anticoagulante sur des échantillons dont la distribution des dimensions des grains est quasi-identique.

II - Préparation de matériaux ayant le Dextrane comme Polymère Support

Synthèse de composés réticulés, insolubles

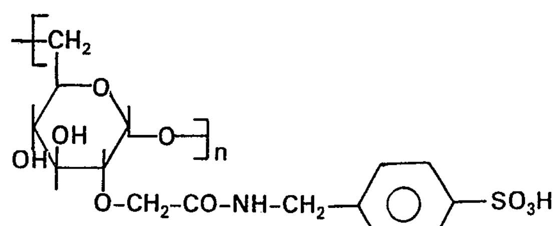

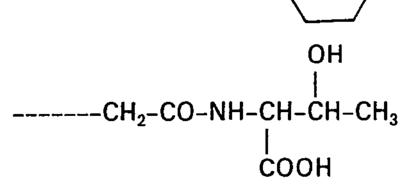

Produit de départ:

Carboxyméthyl-Séphadex (4,0-4,5 meq/g) mis sous forme acide.

1) Fixation de la benzylamine

- dans un ballon de 250 cm³, on disperse sous

agitation 5 g de carboxyméthyl-Séphadex dans 60 m³ d'eau;

- on ajoute, lentement une solution de 20 g d'EEDQ dissous dans 160 ml d'éthanol absolu et on laisse agir une demi-heure;
- on ajoute 4,4 cm³ de benzylamine;
- on laisse le mélange sous agitation 24 heures;
- on filtre pour récupérer le produit;
- on lave abondamment à l'eau distillée, puis à l'éthanol absolu et encore une fois à l'eau;
- on sèche à l'étuve sous vide à 40°C pendant 24 heures,

on fixe environ 2 à 2,6 meq/g de benzylamine.

2) Fixation de la thréonine

Dans un ballon de 250 cm³, 6 g du produit précédent sont dispersés dans 60 cm³ d'eau distillée.

- on ajoute lentement une solution de 20 g d'EEDQ dissous dans 160 ml d'éthanol absolu et on laisse agir une demi-heure;
- on ajoute ensuite le mélange suivant:

3 g de thréonine
1,1 g de soude
20 cm³ d'eau distillée
et on laisse sous agitation pendant 24 heures;

- on récupère ensuite le produit comme décrit en 1);

on fixe environ 0,6 meq/g de thréonine.

3) Sulfonation

- 5 g du produit précédent sont dispersés dans 500 cm³ de nitrométhane sous agitation:
- on ajoute lentement 2,8 cm³ d'acide chlorosulfonique (quantité correspondant à environ 3 fois la quantité de benzylamine);
- on laisse agir 2 heures;
- on récupère le produit par filtration;
- on lave abondamment au nitrométhane puis à l'eau distillée;
- on met le produit obtenu dans une solution de soude 1 M pendant 2 heures afin de l'hydrolyser totalement;
- on récupère le produit par filtration;
- on lave abondamment à l'eau distillée, et
- on sèche à l'étuve sous vide à 40°C pendant 24 heures.

Si l'on opère en présence d'un excès plus important d'acide chlorosulfonique (>4 fois la quantité de benzylamine), on obtient un produit soluble.

4) Lavage et conditionnement du produit

Afin d'éliminer totalement les réactifs de synthèse, le produit subit successivement deux lavages avec une solution 1,5 M de chlorure de sodium, puis deux lavages avec une solution molaire de citrate trisodique.

Il est ensuite conditionné à pH 7,3-7,35 par trois lavages successifs au tampon de MICHAELIS.

Le produit est enfin lavé abondamment à l'eau distillée et séché à l'étuve sous vide à 40°C pendant 24 heures.

III - Tests de coagulation

Le plasma pauvre en plaquettes (PPP) est préparé à partir de plasma humain frais par centrifugation (4°C - 10 000 g - 30 minutes). Il est stocké à −20°C, dégelé par petites quantités et maintenu alors à 4°C.

Le fibrinogène utilisé est du fibrinogène bovin purifié (Behring) à une concentration de 6 g/l dans NaCl à 0,85%. La solution est faite juste avant l'emploi et conservée à 37°C.

La thrombine (Roche, 50 NIH/mg), l'antagoniste de l'héparine [polybrène ou polylysine (Sigma)] sont dilués extemporanément dans du tampon de MICHAELIS et conservés à 4°C (pour la thrombine) ou à la température ambiante (pour le polybrène et la polylysine). La reptilase (Stago) est diluée dans l'eau distillée et conservée à 37°C. L'antithrombine III (Kabi) est en solution dans l'eau distillée et conservée à 4°C.

Les polymères sont en suspension dans du tampon de MICHAELIS additionné de 1 g/l d'un agent émulsifiant (Lensex TA 01 - NP 40 Shell).

Tous les temps de coagulation sont déterminés à 37°C en tube de verre par observation directe.

IV - Etude de l'activité anticoagulante

L'activité anticoagulante des résines a été étudiée par détermination des temps de thrombine de plasma décalcifié et pauvre en plaquettes (PPP) ou de solution de fibronogène en présence de suspension de la résine à étudier en concentration variable. Des études préliminaires de plusieurs résines PS-$SO_3$ contenant des proportions variées de groupes -$SO_3$Na, ont montré que le temps de thrombine ne dépend que de la quantité de groupes -$SO_3$Na présents dans un volume donné de PPP au moment du test. En conséquence, pour pouvoir comparer directement l'activité anticoagulante de différentes résines, les temps de thrombine ont été portés en fonction de la quantité de résine exprimée en concentration de -$SO_3$Na.

Les résultats sont représentés sur la figure 1. On a porté en ordonnée le temps de thrombine en secondes, et en abscisse la concentration de la suspension de polymère (exprimée en nombre de groupes $SO_3^-$/ml) pour 30 U/ml de thrombine (figure 1a), 50 U/ml de thrombine (figure 1b) et 75 U/ml de thrombine (figure 1c).

Les courbes 1 correspondent à la résine PS-$SO_3$ avec du PPP.

Les courbes 2 correspondent à la résine PS-$SO_2$-Glu avec du PPP.

Les courbes 3 correspondent à la résine PS-$SO_3$ avec la solution de fibrinogène.

Les courbes 4 correspondent à la résine PS-$SO_2$-Glu avec la solution de fibrinogène.

Une suspension de 0,1 ml de polymère (de concentration variable) est incubée avec 0,2 ml de PPP (courbes continues sur la figure 1) ou d'une solution de fibrinogène (courbes en traits discontinus sur la figure 1) pendant 30 minutes à 37°C. On ajoute alors 0,1 ml de thrombine et on mesure le temps de coagulation.

En examinant la figure 1, on constate immédiatement que l'effet anticoagulant de la résine PS-$SO_2$-Glu est plus grand que celui de la résine

$PS-SO_3$. Cette différence ne peut pas être attribuée à la différence de taille des grains ou de surface spécifique, celle-ci étant beaucoup trop faible pour expliquer les écarts observés sur les temps de coagulation.

Dans tous les cas, les contrôles supplémentaires suivants ont été faits:

a) détermination du temps de thrombine du PPP ou de la solution de fibrinogène en l'absence de polymère (temps témoins),

b) détermination du temps de thrombine sur le surnageant obtenu par centrifugation du PPP préincubé avec le polymère. Dans tous les cas, ce temps a été trouvé très voisin du temps témoin. De plus, pour vérifier que l'augmentation du temps de thrombine n'est-pas due à une altération du fibrinogène, on a également déterminé les temps de coagulation par la reptilase, de PPP ou d'une solution de fibrinogène incubés avec le polymère. Aucune différence significative n'a pu être mise en évidence entre les temps de reptilase obtenus en présence ou en l'absence du polymère, ainsi que cela ressort des résultats représentés sur la figure 2. On a tracé sur cette figure 2 le temps de coagulation en secondes en fonction de la concentration de la suspension de polymère (exprimée en nombre de groupes de $SO_3^-$/ml). La suspension de polymère (0,1 ml) est incubée avec 0,2 ml de PPP pendant 30 minutes à 37°C. On ajoute alors 0,1 ml de thrombine à 9 U/ml (courbe continue) ou de reptilase (courbe en traits discontinus). La concentration de la thrombine a été choisie pour donner un temps témoin identique à la reptilase, à savoir 20 secondes. Les courbes 1 correspondent au $PS-SO_3$ et les courbes 2 à un polymère conforme à l'invention ($PS-SO_2$-Glu).

Les courbes obtenues avec le plasma, pour différentes concentrations de thrombine ont également été transposées en quantité de thrombine inactivée en fonction de la quantité de résine présente dans le test. En effet, en première approximation, on peut considérer qu'un temps de thrombine allongé traduit l'inactivation d'une fraction de la thrombine initiale et déterminer cette fraction en comparant le temps de coagulation obtenu avec une courbe de temps témoins mésurés avec des quantités de thrombine variables. La figure 3 montre les courbes ainsi obtenues.

Les courbes 1 correspondent au polymère $PS-SO_3$.

Les courbes 2 correspondent au polymère conforme à l'invention $PS-SO_2$-Glu.

Les courbes 3 correspondent aux surnageants (PPP + $PS-SO_3$ et PPP + $PS-SO_2$-Glu).

On a porté en ordonnée la thrombine inactivée en U/ml et en abscisse la concentration de la suspension de polymère exprimée en nombre de groupes $SO_3^-$/ml. La quantité de thrombine inactivée a été évaluée à partir d'une courbe d'étalonnage de temps témoins (0,2 ml de PPP sont incubés avec 0,1 ml de tampon de MICHAELIS à 37°C. On ajoute 0,1 ml de thrombine - de concentration variable - et on mesure le temps de coagulation). Les temps de thrombine, mesurés après incubation de PPP avec les suspensions de polymère, sont reportés sur cette courbe d'étalonnage, ce qui permet d'avaluer la concentration d'enzyme active, donc la quantité correspondante de thrombine inactivée.

Les courbes représentées sur les figures 3a, 3b, 3c, montrent clairement que la proportion de thrombine inactivée augmente avec la quantité de polymère présente dans le test. Par ailleurs, les mesures de temps de thrombine effectuées sur les surnageants (cf. courbes 3) indiquent qu'il n'y a alors aucune inactivation de la thrombine. Ces résultats montrent que l'effet anticoagulant du polymère ne s'exerce que tant que ce dernier est présent dans le PPP.

La comparaison des temps de thrombine obtenus dans les mêmes conditions pour le PPP et la solution de fibrinogène (cf. figures 1 et 3) montre que l'effet anticoagulant du polymère ne se manifeste qu'en présence d'un facteur plasmatique absent de la solution de fibrinogène.

L'ensemble de ces résultats suggère que l'activité anticoagulante de ces polymères est une activité antithrombique impliquant l'antithrombine III comme cofacteur plasmatique. En d'autres termes, ces polymères sont des matériaux anticoagulants «héparine libre».

V - Etablissement de la loi générale de variation entre la quantité de thrombine inactivé et la quantité de résine $PS-SO_2AA$ présenté

L'activité anticoagulante des différents échantillons a été étudiée par détermination des temps de thrombine de PPP incubé avec des quantités croissants de polymère conforme à l'invention. Au préalable, pour vérifier que l'activité anticoagulante de chaque résine est analogue à celle des échantillons décrits dans le paragraphe IV ci-dessus et dans le paragraphe IX ci-après, les temps de reptilase de PPP incubé avec le polymère et les temps de thrombine de la solution de fibrinogène incubée avec le polymère ont été déterminés. Ils sont tous sensiblement égaux aux temps témoins correspondants (déterminés en l'absence de polymère).

L'allongement du temps de thrombine correspondant à l'inactivation d'une fraction de la thrombine initiale, la comparaison entre une courbe d'étalonnage et le temps de coagulation déterminé en présence d'une certaine quantité de résine, permet de déterminer la quantité de thrombine inactivée par cette quantité de résine présente dans le PPP. La figure 4 montre la variation de la quantité de thrombine inactivée en fonction de la concentration de la suspension de résine pour une série de composés contenant de la proline en proportion variable par rapport aux groupes sulfonate. La concentration de la suspension de résine est ici exprimée en milliéquivalents de groupes $-SO_3Na$ par millilitre de suspension, ce qui permet de comparer directement l'effet des groupes proline et montre que l'activité anticoagulante augmente avec le taux de ces groupes.

La figure 4 représente en effet la quantité de thrombine inactivée en fonction de la concentration de la suspension de polymère PS-$SO_2$-proline (exprimée en meq-$SO_3Na$/ml)

pour 2,59 meq de proline par g de polymère: (courbe 1)
pour 2,09 meq de proline par g de polymère: (courbe 2)
pour 1,62 meq de proline par g de polymère: (courbe 3)
pour 0,93 meq de proline par g de polymère: (courbe 4)

Une même série de courbes a été obtenue pour chaque acide aminé.

La figure 5 montre la variation de la quantité de thrombine inactivée en ordonnée, en fonction de la concentration de polymère (en mg/ml) pour une résine portant des groupes acide glutamique et pour quatre concentrations de thrombine:

- 12 U/ml (courbe 1)
- 30 U/ml (courbe 2)
- 50 U/ml (courbe 3)
- 75 U/ml (courbe 4)

Les mêmes courbes ont été établies pour tous les autres produits conformes à l'invention, préparés, et on a constaté que la loi de variation est dans tous les cas la même. Cette variation est tout d'abord linéaire, ce qui montre que la quantité de thrombine inactivée est proportionnelle à la quantité de polymère présent quelle que soit la concentration de thrombine utilisée. Cependant, pour atteindre 100% d'inactivation, un excès de polymère est toujours nécessaire. L'extrapolation de la tangente à l'origine permet pour chaque polymère, de déterminer la quantité de résine théoriquement nécessaire pour inactiver totalement une certaine quantité de thrombine. Ceci résulte clairement de la figure 6 où les grandeurs ont été portées en coordonnées réduites: en ordonnée, la thrombine inactivée en % du total, et en abscisse la quantité de polymère en % de la quantité théoriquement nécessaire pour inactiver toute la thrombine présente.

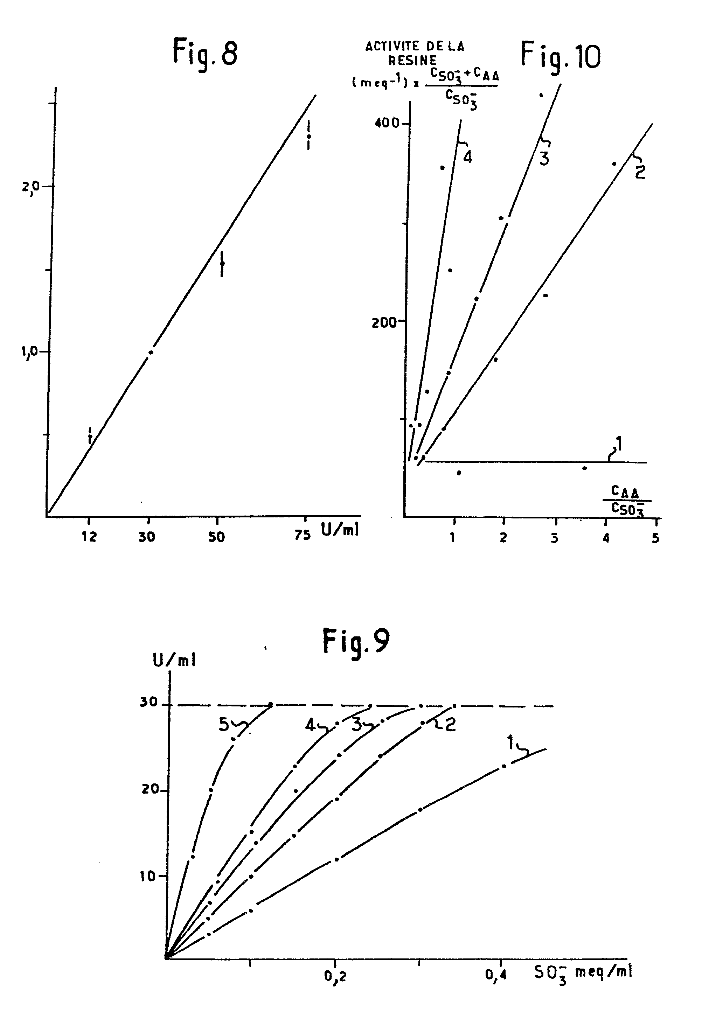

La figure 7 permet de vérifier que cette loi de proportionnalité est générale. En effet, chaque point correspond ici à la quantité de polymère nécessaire pour inactiver 30 unités de thrombine en fonction de la quantité du même polymère nécessaire pour inactiver 12 unités de thrombine. Tous ces points sont sensiblement alignés et très voisins de la droite de pente 30/12 correspondant à la proportionnalité, ce qui montre que la loi de variation est valable pour tous les amino-acides étudiés. Il est alors possible de déterminer une activité pour chaque résine comme l'inverse de la quantité de résine nécessaire pour inactiver 30 unités de thrombine. En utilisant cette valeur comme référence, on peut alors montrer que la loi de proportionnalité est valable pour des concentrations de thrombine variant de 12 à 75 unités/ml, comme le montre la figure 8 où on a porté en abscisse la concentration de la thrombine et en ordonnée le rapport:

Polymère nécessaire pour inactiver X unités de thrombine.

Polymère nécessaire pour inactiver 30 unités de thrombine.

En résumé, il résulte clairement des figures 7 et 8:

- que l'on arrive à inactiver toute la thrombine présente à condition d'ajouter une quantité suffisante de polymère conforme à l'invention,
- que la même loi d'inactivation est suivie par tous les produits conformes à l'invention, quel que soit l'aminoacide (AA) et quelle que soit la quantité de cet AA présente dans la résine.

VI - Cas des diamino-acides (lysine)

On a représenté sur la figure 9, les courbes de variation de la quantité de thrombine inactivée (en ordonnée en U/ml) en fonction de la quantité de résine présente dans le test.

La courbe 1 concerne le polymère PS-$SO_2$-Lysine

La courbe 2 concerne le polymère sulfoné PS-$SO_3$

La courbe 3 concerne le polymère PS-$SO_2$-Proline

La courbe 4 concerne le polymère PS-$SO_2$-$\alpha$ Z Lysine

La courbe 5 concerne le polymère PS-$SO_2$-Glutamique

Z étant le groupe électroattracteur benzyloxycarbonyle.

La quantité de résine étant exprimée en concentration de groupes sulfonate par millilitre de suspension pour toutes les résines, les courbes supérieures à celle d'une résine ne contenant que des groupes sulfonate (courbe 2) indiquent que l'amino-acide correspondant confère à la résine sur laquelle il est fixé, une activité anticoagulante supplémentaire par rapport à celle de ses seuls groupes sulfonate. C'est le cas de tous les amino-acides sauf la lysine. Dans ce cas, au contraire, la courbe est inférieure à celle d'une résine ne contenant que des groupes sulfonate (PS-$SO_3$), c'est-à-dire que la présence de la lysine sur la résine revient à «neutraliser» une partie de l'activité anticoagulante des sites sulfonate. Ceci est à rapprocher de l'effet inhibiteur, décrit au paragraphe IX ci-après, observé avec la polylysine dont les sites amino- des chaînes latérales, protonés à pH 7,35, neutralisent les sites négatifs, notamment sulfonate, d'une résine PS-$SO_3$ ou PS-$SO_2$AA, ou de l'héparine.

Par contre, quand on a bloqué une fonction amine de la lysine (courbe 4), cette neutralisation n'est plus possible et le polymère retrouve pleinement ses propriétés.

VII - Cas des résines PS-$CH_2$-AA (l'acide amine est lie aux chaînes macromoleculaires par l'intermediaire du pont -$CH_2$-)

Cette étude a porté sur trois résines contenant respectivement de la proline, de l'hydroxyproline

et de l'alanine (cf. Tableau I: composés 11, 12 et 13). Dans chaque cas, les temps de thrombine de PPP incubé avec des quantités variables de résine ont été déterminés. Ils ont toujours été trouvés sensiblement égaux au temps témoin, qu'elle que soit la quantité de résine présente.

VIII - Evaluation de l'activité propre de chaque type de substituant

A partir de l'activité de chaque résine définie précédemment par rapport à 30 unités de thrombine, on peut déduire l'activité rapportée à l'unité de thrombine (cf. Tableau II). Si les effets des différents substituants de la résine sont additifs, l'activité «a» peut s'exprimer par la relation (1) ci-après:

$$a(C_{SO_3^-} + C_{AA}) = (a_{SO_3^-} X C_{SO_3^-}) + (a_{AA} X C_{AA}) \quad (1)$$

où $a_{SO_3}$ - et $a_{AA}$ sont les activités respectivement d'un groupe sulfonate et d'un groupe $-SO_2$-amino-acide, $C_{SO_3}$ - et $C_{AA}$ étant leurs concentrations respectives.

La figure 10 montre les droites obtenues en portant l'activité de la résine «a», multipliée par le rapport du nombre total de sites et du nombre de sites sulfonate

$$\frac{C_{SO_3^-} + C_{AA}}{C_{SO_3^-}} \quad \text{(en ordonnée)}$$

en fonction du rapport des nombres de sites $-SO_2$-amino-acides et sulfonate

$$\frac{C_{AA}}{C_{SO_3^-}} \quad \text{(en abscisse)}$$

Ces droites ont une ordonnée à l'origine commune égale à l'activité d'un groupe sulfonate ($a_{SO_3}$-) et des pentes égales aux activités des groupes amino-acides ($a_{AA}$).

La droite 1 correspond au polymère PS-$SO_2$-But-$NH_2$

La droite 2 correspond aux polymères

PS-$SO_2$-alanine
PS-$SO_2$-phénylalanine

La droite 3 correspond aux polymères

PS-$SO_2$-Z Lysine
PS-$SO_2$-hydroxyproline
PS-$SO_2$-méthionine
PS-$SO_2$-proline
PS-$SO_2$-thréonine

La droite 4 correspond au polymère PS-$SO_2$-Acide glutamique.

Les activités des différents types de substituants sont regroupées dans le Tableau II et le Tableau III ci-après:

A - Polymère support: Polystyrène

Tableau II

| Substituant | Coefficient d'activité en $(meq)^{-1}$ $uTh^{-1}$ | Substituant | Coefficient |
|---|---|---|---|
| $-SO_2$ASP<br>$-SO_2$Glu | 550<br>370 | $SO_2$ZLys | 130 |
| $-SO_2$ε-amino-caproïque<br>$-SO_2$β-alanine | 200 à 300<br><br>$-SO_2$-Phe | $-SO_2$-Ala | Compris entre 60-80 |
| $-SO_2$OH Prol | compris entre 120 et 150 | $-SO_3'$ | 60 |
| $-SO_2$Prol<br>$-SO_2$Met<br>$-SO_2$Thr | | $-SO_2$But $NH_2$ | 0 |

On voit clairement que:

- l'activité de la butylamine est nulle, ce qui semble indiquer que le groupe sulfamide seul n'est pas suffisant pour conférer à la résine une activité antithrombique et que les substituants dépourvus de fonction acide carboxylique n'ont aucun effet sur cette activité.
- Les groupes alanine et phénylalanine ont une activité comprise entre 60 et 80 $meq^{-1}$, voisine de celle du groupe sulfonate, 60 $meq^{-1}$, ce qui pourrait indiquer qu'une chaîne latérale hydrocarbonée n'a pratiquement aucun rôle et que l'activité de la fonction carboxylique de l'amino-acide est du même ordre de grandeur que celle du groupe sulfonate. La faible différence entre l'alanine et la phénylalanine s'explique probablement par le caractère hydrophobe plus marqué de la chaîne latérale de la phénylalanine par rapport à l'alanine.
- La lysine dont la fonction amine de la chaîne latérale est substituée par un groupe attracteur d'électrons (groupe Z) a une activité positive, mais difficile à définir avec précision dans la mesure où il n'a pas été possible de synthétiser des résines contenant plus de groupes amino-acide que de groupes sulfonate. Le même problème s'est posé pour la lysine, mais dans ce cas, l'activité, très mal définie également, est négative.
- Les amino-acides dont la chaîne latérale

contient un hétéroatome, comme l'hydroxyproline, la thréonine et la méthionine, ont une activité plus élevée comprise entre 120 et 150 $meq^{-1}$.

La proline semble également pouvoir être rattachée à ce groupe. Il faut noter que deux de ces amino-acides, proline et hydroxyproline, fixés sur le polystyrène par un groupe méthylène (résine $PS-CH_2AA$) ne confèrent aucune activité anticoagulante au materiau.

- Les acides dicarboxyliques, comme l'acide glutamique ou l'acide aspartique, ont une activité plus élevée que tous les autres α-amino-acides, probablement liée à leurs deux fonctions carboxyliques.

- Les acides aminés dans lesquels la fonction amine et la fonction carboxylique sont séparées par plusieurs groupes méthylène, comme par exemple la β-alanine, ou l'acide ε-aminocaproïque, ont une activité d'autant plus élevée que le nombre de groupes méthylène est plus grand, probablement liée à un effet de «bras» de la chaîne carbonée rendant plus accessible la fonction carboxylique.

L'ensemble de ces résultats permet de tirer un certain nombre de conclusions. Le groupe sulfamide n'est pas suffisant pour qu'il y ait une activité anticoagulante; cependant, il semble qu'il soit nécessaire puisque les résines $PC-CH_2-AA$ n'ont aucune activité.

Les groupes sulfonate, en revanche, sont suffisants pour qu'il y ait activité anticoagulante (cf. également le travail de GREGOR précédemment cité), mais celle-ci est faible à très faible, au maximum 15% de celle de l'héparine, quand tous les sites du polymère (PS) sont substitués par des groupes $SO_3^-$.

Par contre, quand la chaîne macromoléculaire porte à la fois des groupes sulfonate, des amino-acides fixés par un pont sulfamide et/ou amide et que les amino-acides ont une chaîne latérale favorable, l'activité anticoagulante du matériau peut être très importante.

B - Polymère support: Dextrane

Tableau III
Etude du temps de coagulation en fonction de la nature de $R_3$ et de la quantité de $SO_3^-$ dans le polymère

| | Nature de $R_3$ | Meq de $SO_3^-$ dans la solution initiale de polymère nécessaire pour avoir le temps de thrombine de 20 secondes (concentration de thrombine 30 U/ml) |
|---|---|---|
| 1 | $-CH_2-$ 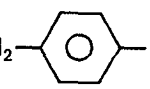 | $17,10^{-3}$ |
| 2 | $-CH_2CO-NH-CH_2-$ 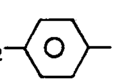 | $2,5.10^{-3}$ |
| 3 | $-CH_2-\underset{\underset{O}{\|}}{C}-NH-CH_2-$ | $100.10^{-3}$ |

Témoin sans polymère: 7 secondes.

Il résulte de la comparaison de ces trois produits que c'est le produit n° 2 qui s'est avéré le meilleur: temps de 20 secondes pour seulement une concentrations de $2,5.10^{-3}$ en meq de $SO_3^-$.

Pour des produits très finement broyés on a trouvé ainsi, à partir de la mesure des temps de thrombine, les coefficients d'activité suivants:

| Substituant | Coefficient d'activité |
|---|---|
| $-CH_2CO-NH-$ $CH_2$ 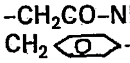 $-SO_3^-$ | $12.10^3$ $meq^{-1}$ u $Th^{-1}$ |
| $-CH_2CO$-Thréonine | $36.10^3$ $meq^{-1}$ u $Th^{-1}$ |

IX - Autres propriétés analogues a l'héparine des composes conformes a l'invention

A - Etude de l'action des inhibiteurs

Les inhibiteurs de l'héparine (polybrène et polylysine) peuvent inhiber totalement l'effet anticoagulant des polymères conformes à l'invention.

Les résultats sont représentés sur la figure 11 annexée (dans laquelle on a porté en ordonnée le temps de thrombine en secondes, et en abscisse la concentration de l'inhibiteur en mg/ml. Le système expérimental est le suivant: la suspension de polymère (0,1 ml) est incubée avec 0,2 ml de PPP et 0,1 ml d'une solution d'inhibiteur (de concentrations variables) pendant 30 minutes à 37°C. On ajoute alors 0,1 ml de thrombine (12 U/ml) et on mesure le temps de coagulation. Les courbes 1 concernent le polybrène et les courbes 2 la polylysine.

La figure 11a représente les résultats obtenus

avec le $PS-SO_2-Glu$ et la figure 11b ceux obtenus avec le polymère $PS-SO_3$. On voit sur la figure 11 que cette inhibition n'est totale qu'à condition qu'il y ait un excès de charges positives de l'inhibiteur par rapport aux charges négatives du polymère, alors que pour l'héparine elle est totale sans excès. Il est à noter également qu'il faut un excès plus grand avec le polybrène qu'avec la polylysine. Ces résultats indiquent qu'une grande proportion des sites négatifs accessibles de la résine, sont impliqués dans son activité anticoagulante.

B - Etude de l'inactivation de la thrombine par les polymères en présence ou en l'absence d'anti-thrombine III

Cette seconde étude a été faite en deux séries:

a) les polymères sont incubés avec de la thrombine ou successivement avec de la thrombine et du polybrène.

b) Les polymères sont incubés avec de la thrombine et de l'antithrombine III ou successivement avec de la thrombine, de l'antithrombine III et du polybrène. Dans tous les cas, le mélange est alors centrifugé et le surnageant est ajouté à du PPP ou à une solution de fibrinogène. On mesure alors le temps de coagulation (cf. Tableau IV ci-après). Les résultats obtenus montrent qu'en l'absence d'antithrombine III, la thrombine est inactivée de façon réversible par le polymère; son activité réapparaît en présence de polybrène. En revanche, en présence d'antithrombine III, l'inactivation de la thrombine par le polymère est irréversible, comme c'est le cas avec l'héparine.

Tableau IV

Etude de l'inanctivation de la thrombine par les polymères en presence ou en l'absence d'anithrombine III

| Echantillon | | Thrombine + tampon (a) * | Thrombine + polybrène (b) * | Thrombine + antithrombine + tampon (c) * | Thrombine + antithrombine + polybrène (d) * |
|---|---|---|---|---|---|
| $PS-SO_3$ | PPP | 70 | 45 | 250 | 64 |
| | Solution de fibrinogène | 110 | 44 | 300 | 60 |
| $PS-SO_2Glu$ | PPP | 112 | 45 | 300 | 65 |
| | Solution de fibrinogène | 150 | 47 | 360 | 62 |

* temps de coagulation en secondes

Système expérimental: une suspension (0,2 ml) de polymère dans du tampon est incubée à 0°C avec 0,2 ml de thrombine à 5 unités/ml (a) (b) ou avec 0,016 ml d'antithrombine III à 25 unités/ml et 0,2 ml de thrombine à 5 unités/ml (c) (d). Après 5 minutes, 0,2 ml de tampon (a) (c) ou 0,2 ml d'une solution de polybrène (40 mg/ml dans du tampon) (b) (d) sont ajoutés. Après 5 minutes à 0°C, la suspension est centrifugée et 0,3 ml de surnageant sont ajoutés à 0,2 ml de PPP ou 0,2 ml de solution de fibrinogène à 37°C. Le temps de coagulation (en sec.) est alors mesuré. Temps témoin: 45 secondes en l'absence d'antithrombine III et 60 secondes en présence d'antithrombine III.

Il résulte de la description qui précède que les produits conformes à la présente invention présentent une activité anticoagulante très marquée: si l'on choisit comme polymère support un polymère réticulé, l'on obtient des produits à action anticoagulante, insolubles, à usage médical ou chirurgical, par exemple. Cette activité anticoagulante à l'état solide est relativement élevée; elle est du même ordre de grandeur que celle de l'héparine elle-même fixée par des liaisons covalentes sur des matériaux polymères - mais contrairement à cette dernière, les produits conformes à l'invention sont remarquablement stables.

**Revendications**

1. Produits insolubles doués de propriétés anticoagulantes, caractérisés en ce qu'ils sont constitués par des polymères, homopolymères ou copolymères réticulés et insolubles choisis parmi le polystyrène et les polysaccharides, comportant dans leur chaîne des groupes substituables sur lesquels sont fixés de façon statistique des groupes X et Y et/ou V, où

X désigne le groupe $-SO_3R_1$ ou $-R_3-SO_3R_1$

$R_1$ étant un atome d'hydrogène ou d'un métal physiologiquement compatible,

$R_3$ étant un groupe $-CH_2-CO-NH-R_4-$ dans lequel $R_4$ représente un radical alkylène, arylène ou alkylarylène substitué ou non

ou $-CH_2$ 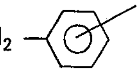 substitué ou non,

Y désigne le groupe $-SO_2-R_2$ ou $-R_3-SO_2-R_2$,

$R_2$ étant le reste d'un acide aminé quelconque dans le cas des polysaccharides et choisi dans le cas du polystyrène dans le groupe qui comprend la méthionine, la cystéine, l'acide cystéique, la proline, l'hydroxyproline, la sérine, la tyrosine, la benzyloxycarbonyl-lysine, la tertiobutyl-oxycarbonyl-lysine, l'acide ε-aminocaproïque, la β-alanine, l'acide γ-amino-n-butyrique, l'acide δ-ami-

no-n-valérique, substitués ou non, lequel acide aminé est lié au pont $-SO_2-$ par sa fonction amine et

V désigne le groupe $-CH_2-CO-NH-CHR-COOH$, R étant la chaîne latérale d'un acide aminé, étant bien entendu que:

a) si X est $-SO_3R_1$, il est obligatoirement accompagné de Y et/ou de V, et

b) V est toujours accompagné de X et/ou de Y.

2. Produits selon la revendication 1, caractérisés en ce que, dans le cas des polysaccharides, les acides aminés sont choisis parmi ceux qui comportent au moins une fonction carboxylique libre.

3. Produits selon la revendication 1, caractérisés en ce que l'acide aminé est un acide aminé dicarboxylique.

4. Produits selon l'une quelconque des revendications 1 à 2, caractérisés en ce que lorsque les acides aminés possèdent plusieurs fonctions amines, toutes sauf une doivent être bloquées par un groupe électroattracteur physiologiquement acceptable.

5. Produits selon la revendication 4, caractérisés en ce que le groupe électroattracteur est avantageusement constitué par un groupe benzyloxycarbonyle ou tertiobutyloxycarbonyle.

6. Produits selon l'une quelconque des revendications 1 à 5, caractérisés en ce que les polymères comportant dans leur chaîne des groupes substituables sur lesquels sont fixés les groupes X et/ou Y et/ou V, sont des polystyrènes.

7. Produits selon l'une quelconque des revendications 1 à 5, caractérisés en ce que les polymères comportant dans leur chaîne des groupes substituables sur lesquels sont fixés des groupes X et/ou Y et/ou V sont des polysaccharides.

8. Produits selon la revendication 7, caractérisés en ce que les polymères sont des dextranes.

9. Procédé de préparation des produits selon l'une quelconque des revendications 1 à 8, caractérisé - lorsqu'on désire obtenir les produits dans lesquels

X = $-SO_3R_1$
Y = $-SO_2-R_2$
et
V est nul

($R_1$ et $R_2$ ayant la même signification que précédemment) - en ce qu'au cours d'une première étape, on prépare un polymère chlorosulfoné POL-$SO_2Cl$ par réaction de l'acide chlorosulfonique sur le polymère, dans un solvant approprié, et en ce qu'au cours d'une deuxième étape, on transforme les groupes $-SO_2Cl$ en groupes $-SO_3Na$ et en groupes $-SO_2AA$ (où AA représente un acide aminé) par réaction avec une quantité appropriée d'un acide aminé en milieu basique.

10. Procédé selon la revendication 9, caractérisé en ce que la sulfonation du polymère réticulé a lieu dans un mélange contenant du dichlorométhane et du nitrométhane, et la réaction avec l'acide aminé a lieu dans un milieu contenant le mélange eau-dioxane.

11. Procédé selon la revendication 10, caractérisé en ce qu'on procède, après la fixation des acides aminés, à un lavage abondant à l'eau suivi de lavages par une solution de NaCl (1,5 M), puis de lavages au citrate de Na (1 M), d'un équilibrage à pH 7,30 environ par plusieurs lavages avec du tampon de MICHAELIS, d'un nouveau lavage à l'eau et enfin d'un séchage.

12. Procédé de préparation de produits selon l'une quelconque des revendications 1 à 9, caractérisé, lorsqu'on désire obtenir les produits dans lesquels

X = $-R_3-SO_3R_1$
V = $-CH_2-CO-NH-CHR-COOH$
et
Y est nul

(R, $R_1$ et $R_3$ ayant la même signification que précédemment), en ce que l'on prépare, au cours d'une première étape, les dérivés carboxyméthylés des polymères, en ce que l'on fixe, au cours de la deuxième étape, les amines appropriées ou le chlorure de benzyle, en ce que l'on fixe les acides aminés au cours de la troisième étape et en ce que l'on procède à la sulfonation au cours de la quatrième étape.

13. Procédé selon la revendication 12, caractérisé en ce que le couplage des amines et/ou des acides aminés sur des dérivés carboxyméthylés de polymères s'effectue à l'aide de N-éthoxycarbonyl-2-éthoxy 1,2-dihydroquinoléine (EEDQ).

14. Procédé selon la revendication 12, caractérisé en ce que l'étape de sulfonation est répétée plusieurs fois si l'on désire augmenter le taux de groupements sulfonates.

15. Procédé de préparation des produits selon l'une quelconque des revendications 1 à 8, caractérisé, lorsqu'on désire obtenir les produits dans lesquels

X = $-R_3-SO_3R_1$
V = $-CH_2-CO-NH-CHR-COOH$
et
Y = $-R_3-SO_2R_2$

(R, $R_1$, $R_2$ et $R_3$ ayant la même signification que précédemment), en ce qu'au cours de la première étape, on procède à la carboxyméthylation, en ce qu'au cours d'une deuxième étape, on fixe les amines appropriées ou le chlorure de benzyle, en ce qu'au cours d'un troisième étape on procède à la chlorosulfonation, en ce qu'au cours d'une quatrième étape on fixe des acides aminés.

16. Objets à usage médical et chirurgical tels que tubes, prothèses cardiovasculaires, cathéters, fils et films en polymères comportant des groupes substituables, rendus anticoagulants par fixation sur leur surface extérieure des groupes X et/ou Y et/ou V, tels que définis dans la revendication 1.

17. Procédé de fabrication des objets selon la revendication 16, caractérisé en ce que l'on façonne un objet de forme et de dimension voulues à partir d'un polymère comportant des groupes

substituables et en ce que l'on fixe chimiquement selon l'une quelconque des revendications 9, 12, 15, les groupes X et/ou Y et/ou V tels que définis dans la revendication 1, en quantités voulues et aussi élevées qu'on le désire, pour obtenir un matériau solide à action anticoagulante.

18. Objets insolubles à usage médical et chirurgical tels que tubes, prothèses, cathéters, fils et films façonnés à partir de produits selon l'une quelconque des revendications 1 à 5.

**Patentansprüche**

1. Unlösliche Produkte mit antikoagulierenden Eigenschaften, dadurch gekennzeichnet, dass sie aus Polymeren, Homopolymeren oder Copolymeren, vernetzt und unlöslich, ausgewählt aus Polystyrol und den Polysacchariden, bestehen, die in ihrer Kette substituierbare Gruppen tragen, an denen in statistischer Weise Gruppen X und Y und/oder V fixiert sind, worin
X die Gruppe $-SO_3R_1$ oder $-R_3-SO_3R_1$ bezeichnet, wobei $R_1$ ein Wasserstoffatom oder ein physiologisch verträgliches Metall ist,
$R_3$ eine Gruppe $-CH_2-CO-NH-R_4-$ ist, worin $R_4$ einen Alkylenrest, Arylenrest oder Alkylarylenrest, substituiert oder unsubstituiert,

oder $-CH_2$ 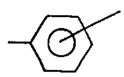 substituiert oder

unsubstituiert, bedeutet,
Y die Gruppe $-SO_2-R_2$ oder $-R_3-SO_2-R_2$ bedeutet, worin $R_2$ den Rest einer beliebigen Aminosäure im Fall von Polysacchariden darstellt und im Fall von Polystyrol ausgewählt ist aus der Gruppe, die das Methionin, das Cystein, die Cysteinsäure, das Prolin, das Hydroxyprolin, das Serin, das Tyrosin, das Benzyloxycarbonyl-Lysin, das tert.-Butyloxycarbonyl-Lysin, die ε-Aminocapronsäure, das β-Alanin, die γ-Amino-n-buttersäure, die δ-Amino-n-valeriansäure, substituiert oder unsubstituiert, umfasst, wobei diese Aminosäure über $-SO_2$-Brücken durch ihre Aminfunktion gebunden ist und
V die Gruppe $-CH_2-CO-NH-CHR-COOH$ bedeutet,
R die Seitenkette einer Aminosäure darstellt, wobei es sich versteht, dass:

a) wenn X die Bedeutung von $-SO_3R_1$ hat, es obligatorisch von Y und/oder V begleitet wird, und

b) V immer von X und/oder Y begleitet wird.

2. Produkte nach Anspruch 1, dadurch gekennzeichnet, dass im Falle von Polysacchariden die Aminosäuren unter solchen ausgewählt sind, die mindestens eine freie Carboxylfunktion tragen.

3. Produkte nach Anspruch 1, dadurch gekennzeichnet, dass die Aminosäure eine Aminodicarbonsäure ist.

4. Produkte nach einem der Ansprüche 1 bis 2, dadurch gekennzeichnet, dass wenn die Aminosäuren mehrere Aminfunktionen aufweisen, sämtliche, ausser einer blockiert sein müssen durch eine physiologisch brauchbare Elektronen anziehende Gruppe.

5. Produkte nach Anspruch 4, dadurch gekennzeichnet, dass die Elektronen anziehende Gruppe vorzugsweise durch eine Benzyloxycarbonylgruppe oder tert.-Butyloxycarbonylgruppe gebildet wird.

6. Produkte nach einem der Ansprüche 1 bis 5, dadurch gekennzeichnet, dass die Polymeren, die in ihrer Kette substituierbare Gruppen tragen, an die die Gruppen X und/oder Y und/oder V fixiert sind, Polystyrole sind.

7. Produkte nach einem der Ansprüche 1 bis 5, dadurch gekennzeichnet, dass die Polymeren, die in ihrer Kette substituierbare Gruppen tragen, an denen die Gruppen X und/oder Y und/oder V fixiert sind, Polysaccharide sind.

8. Produkte nach Anspruch 7, dadurch gekennzeichnet, dass die Polymeren Dextrane sind.

9. Verfahren zur Herstellung der Produkte nach einem der Ansprüche 1 bis 8, dadurch gekennzeichnet, dass man, wenn man Produkte erhalten will, worin

$X = -SO_3R_1$
$Y = -SO_2-R_2$
und
$V = $ Null

(wobei $R_1$ und $R_2$ die gleiche Bedeutung wie vorstehend haben) im Verlauf einer ersten Stufe ein chlorsulfoniertes Polymeres $POL-SO_2Cl$ herstellt durch Reaktion von Chlorsulfonsäure mit dem Polymeren in einem geeigneten Lösungsmittel, und dass man im Verlauf einer zweiten Stufe die Gruppen $-SO_2Cl$ in Gruppen $-SO_3Na$ und in Gruppen $-SO_2AA$ (worin AA eine Aminosäure darstellt) umwandelt, durch Reaktion mit einer geeigneten Menge einer Aminosäure, in basischem Milieu.

10. Verfahren nach Anspruch 9, dadurch gekennzeichnet, dass die Sulfonierung des vernetzten Polymeren in einem Gemisch erfolgt, das Dichlormethan und Nitromethan enthält, und dass die Reaktion mit der Aminosäure in einem Milieu erfolgt, das ein Gemisch Wasser-Dioxan enthält.

11. Verfahren nach Anspruch 10, dadurch gekennzeichnet, dass man nach der Fixierung der Aminosäure eine reichliche Wäsche mit Wasser und anschliessend Wäschen mit einer Lösung von NaCl (1,5 M) anschliessend Wäschen mit Na-Zitrat (1 M), eine Einstellung auf den pH-Wert von etwa 7,30 mit mehreren Wäschen mit MICHAELIS-Puffer, und eine neue Wäsche mit Wasser und schliesslich eine Trocknung durchführt.

12. Verfahren zur Herstellung der Produkte nach einem der Ansprüche 1 bis 9, dadurch gekennzeichnet, dass man, falls man Produkte erhalten will, worin

$X = -R_3-SO_3R_1$
$V = -CH_2-CO-NH-CHR-COOH$
und
$Y = $ Null

(wobei R, $R_1$ und $R_3$ die gleiche Bedeutung wie vorstehend haben), im Verlauf einer ersten Stufe die carboxymethylierten Derivate der Polymeren herstellt, in einer zweiten Stufe die geeigneten Amine oder Benzylchlorid fixiert und in einer dritten Stufe die Aminosäuren fixiert und in einer vierten Stufe die Sulfonierung durchführt.

13. Verfahren nach Anspruch 12, dadurch gekennzeichnet, dass man die Kupplung der Amine und/oder Aminosäuren an die carboxymethylierten Derivate der Polymeren mit Hilfe von N-Ethoxycarbonyl-2-ethoxy-1,2-dihydrochinoleinsäure (EEDQ) durchführt.

14. Verfahren nach Anspruch 12, dadurch gekennzeichnet, dass die Sulfonierungsstufe mehrfach wiederholt wird, wenn der Sulfonatgruppengehalt erhöht werden soll.

15. Verfahren zur Herstellung der Produkte nach einem der Ansprüche 1 bis 8, dadurch gekennzeichnet, dass man, wenn man Produkte herstellen will, worin

$X = -R_3-SO_3R_1$
$V = -CH_2-CO-NH-CHR-COOH$
und
$Y = -R_3-SO_2R_2$

(wobei R, $R_1$, $R_2$ und $R_3$ die gleiche Bedeutung wie vorstehend haben), im Verlauf einer ersten Stufe eine Carboxymethylierung durchführt, im Verlauf einer zweiten Stufe die geeigneten Amine oder Benzylchlorid fixiert, im Verlauf einer dritten Stufe die Chlorsulfonierung durchführt und im Verlauf einer vierten Stufe die Aminosäuren fixiert.

16. Gegenstände zur medizinischen und chirurgischen Verwendung, wie Rohre, cardiovaskuläre Prothesen, Katheter, Fäden und Folien, aus Polymeren, die substituierbare Gruppen tragen, die durch Fixierung an ihre äussere Oberfläche von Gruppen X und/oder Y und/oder V, wie in Anspruch 1 definiert, antikoagulierend gemacht wurden.

17. Verfahren zur Herstellung der Gegenstände nach Anspruch 16, dadurch gekennzeichnet, dass man einen Gegenstand mit gewünschter Form und Abmessung aus einem Polymeren formt, das substituierbare Gruppen trägt, und man chemisch nach einem der Ansprüche 9, 12, 15, die Gruppen X und/oder Y und/oder V, wie in Anspruch 1 definiert, in gewünschten und so hoch wie gewünschten Mengen, fixiert, um ein festes Material mit antikoagulierender Wirkung zu erhalten.

18. Unlösliche Gegenstände zur medizinischen und chirurgischen Verwendung, wie Rohre, Prothesen, Kathether, Fäden und Folien, geformt aus Produkten gemäss einem der Ansprüche 1 bis 5.

## Claims

1. Insoluble products possessing anti-coagulating properties, characterized in that they consist of crosslinked and insoluble polymers, homopolymers or copolymers chosen from the group comprising polystyrene and polysaccharides, containing, in their chain, substitutable groups to which groups X and Y and/or V are randomly attached, where

X denotes the group $-SO_3R_1$ or $-R_3-SO_3R_1$,
$R_1$ being a hydrogen atom or a physiologically compatible metal and
$R_3$ being a group $-CH_2-CO-NH-R_4-$, in which $R_4$ represents a substituted or unsubstituted alkylene, arylene or alkylarylene radical,

or substituted or unsubstituted $-CH_2$ 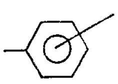 ,

Y denotes the group $-SO_2-R_2$ or $-R_3-SO_2-R_2$,
$R_2$ being the radical of any amino acid in the case of polysaccharides and being chosen, in the case of polystyrene, from the group comprising methionine, cysteine, cysteic acid, proline, hydroxyproline, serine, tyrosine, benzyloxycarbonyllysine, tert.-butoxycarbonyllysine, ε-aminocaproic acid, β-alanine, γ-amino-n-butyric acid and δ-amino-n-valeric acid, which are substituted or unsubstituted, the said amino acid being bonded to the $-SO_2$-bridge by its amine group, and
V denotes the group $-CH_2-CO-NH-CHR-COOH$,
R being the side chain of an amino acid,
it being understood that:

a) if X is $-SO_3R_1$, it is necessarily accompanied by Y and/or V, and

b) V is always accompanied by X and/or Y.

2. The products according to Claim 1, characterized in that, in the case of polysaccharides, the amino acids are chosen from those containing at least one free carboxyl group.

3. The products according to Claim 1, characterized in that the amino acid is a dicarboxylic amino acid.

4. The products according to either one of Claims 1 and 2, characterized in that, if the amino acids possess several amine groups, all but one of these amine groups must be blocked by a physiologically acceptable electron-attracting group.

5. The products according to Claim 4, characterized in that the electron-attracting group advantageously consists of a benzyloxycarbonyl or tert.-butoxycarbonyl group.

6. The products according to any one of Claims 1 to 5, characterized in that the polymers containing, in their chain, substitutable groups to which the groups X and/or Y and/or V are attached are polystyrenes.

7. The products according to any one of Claims 1 to 5, characterized in that the polymers containing, in their chain, substitutable groups to which groups X and/or Y and/or V are attached are polysaccharides.

8. The products according to Claim 7, characterized in that the polymers are dextrans.

9. A method for the preparation of the products according to any one of Claims 1 to 8, characterized in that - if it is desired to obtain the products in which

$X = -SO_3R_1$,
$Y = -SO_2-R_2$ and
$V =$ zero

($R_1$ and $R_2$ having the same meanings as above) – in a first step a chlorosulphonated polymer, POL-$SO_2Cl$, is prepared by reacting chlorosulphonic acid with the polymer in a suitable solvent, and in a second step the $-SO_2Cl$ groups are converted to $-SO_3Na$ groups and to $-SO_2AA$ groups (in which AA represents an amino acid) by reaction with a suitable quantity of an amino acid in a basic medium.

10. The method according to Claim 9, characterized in that the sulphonation of the crosslinked polymer takes place in a mixture containing methylene chloride and nitromethane and the reaction with the amino acid takes place in a medium containing a water/dioxane mixture.

11. The method according to Claim 10, characterized in that, after attachement of the amino acids, the products are washed copiously with water, then several times with a solution of NaCl (1.5 M) and then several times with Na citrate (1 M), followed by equilibration to a pH of about 7.30 by washing several times with MICHAELIS buffer, by washing again with water and finally by drying.

12. A method for the preparation of products according to any one of Claims 1 to 9, characterized in that – if it is desired to obtain the products in which

$X = -R_3-SO_3R_1$,
$V = -CH_2-CO-NH-CHR-COOH$ and
Y is zero

(R, $R_1$ and $R_3$ having the same meanings as above) – in a first step the carboxymethylated derivatives of the polymers are prepared, in the second step the appropriate amines or benzyl chloride are attached, in the third step the amino acids are attached and in the fourth step sulphonation is carried out.

13. The method according to Claim 12, characterized in that the coupling of the amines and/or amino acids with carboxymethylated derivatives of polymers is carried out with the aid of N-ethoxycarbonyl-2-ethoxy-1,2-dihydroquinoline (EEDQ).

14. The method according to Claim 12, characterized in that the sulphonation step is repeated several times if it is desired to increase the proportion of sulphonate groups.

15. A method for the preparation of the products according to any one of Claims 1 to 8, characterized in that – if it is desired to obtain the products in which

$X = -R_3-SO_3R_1$,
$V = -CH_2-CO-NH-CHR-COOH$ and
$Y = -R_3-SO_2R_2$

(R, $R_1$, $R_2$ and $R_3$ having the same meanings as above) – in the first step carboxymethylation is carried out, in a second step the appropriate amines or benzyl chloride are attached, in a third step chlorosulphonation is carried out and in a fourth step amino acids are attached.

16. Articles for medical and surgical use, such as tubes, cardiovascular prostheses, catheters, threads and films, made of polymers containing substitutable groups, which have been rendered anti-coagulating by attachment of the groups X and/or Y and/or V, such as defined in Claim 1, to their outer surface.

17. A method for the manufacture of the articles according to Claim 16, characterized in that an article of desired shape and size is made from a polymer containing substitutable groups, and in that the groups X and/or Y and/or V, such as defined in Claim 1, are chemically attached, according to any one of Claims 9, 12 and 15, in the required quantities, which are as large as desired, to give a solid material which an anticoagulating action.

18. Insoluble articles for medical and surgical use, such as tubes, prostheses, catheters, threads and films, made from products according to any one of Claims 1 to 5.

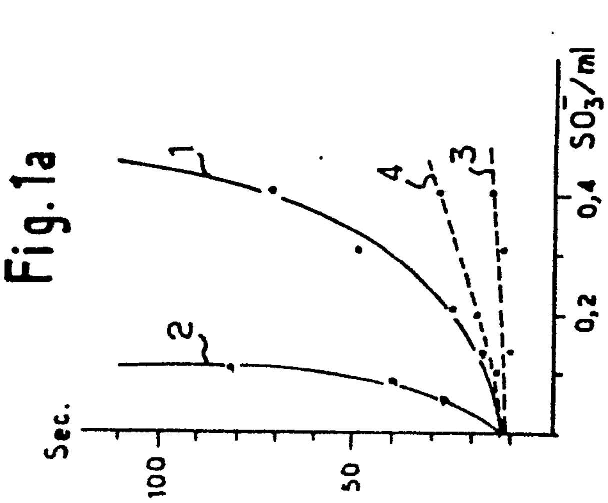


Fig. 1a

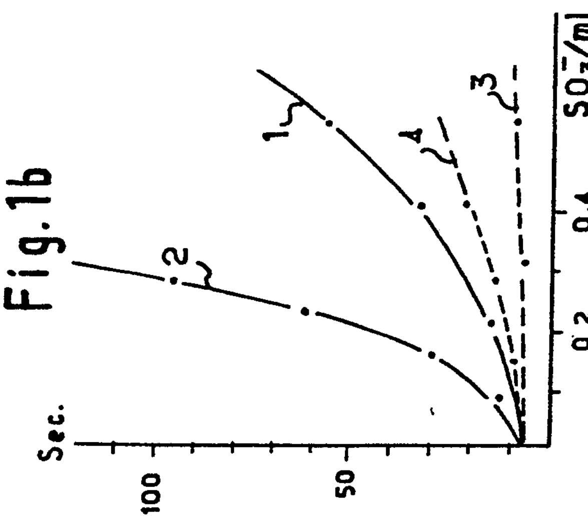


Fig. 1b

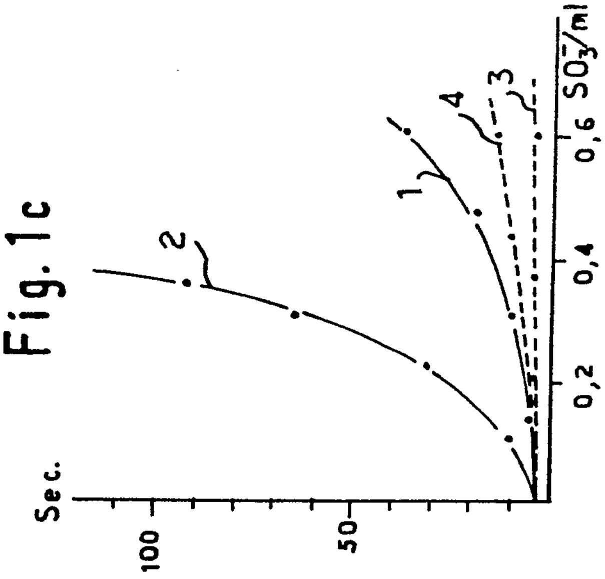


Fig. 1c

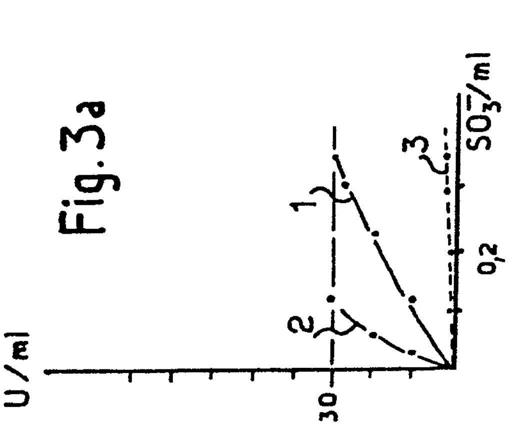


Fig. 3a

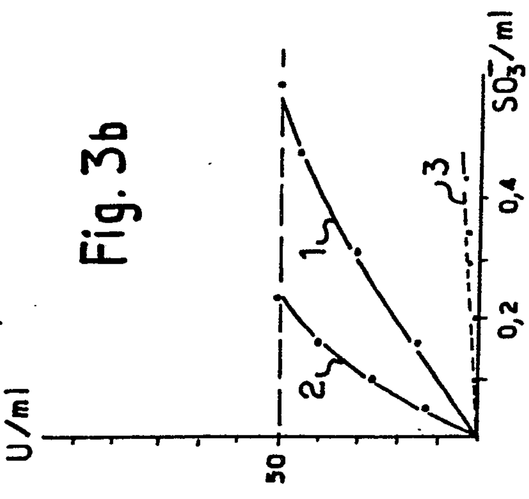


Fig. 3b

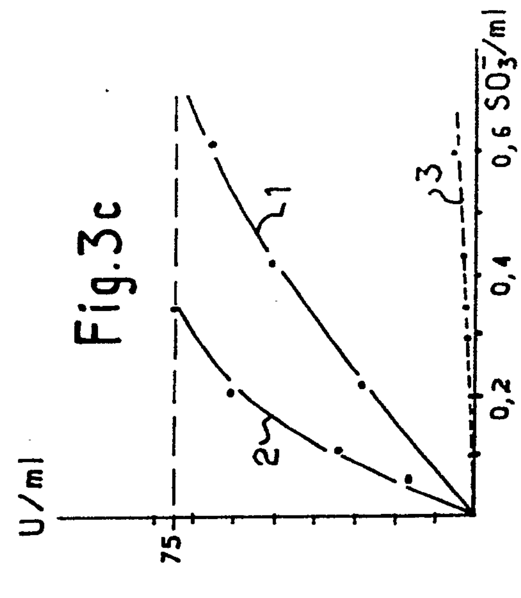


Fig. 3c

Fig. 2

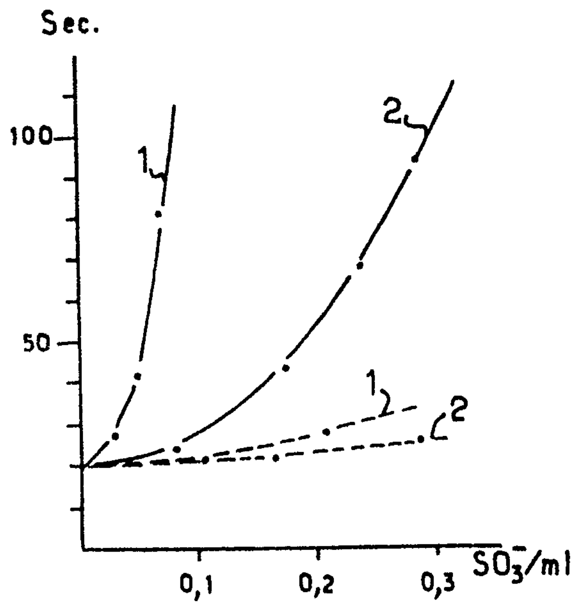


Fig.11a

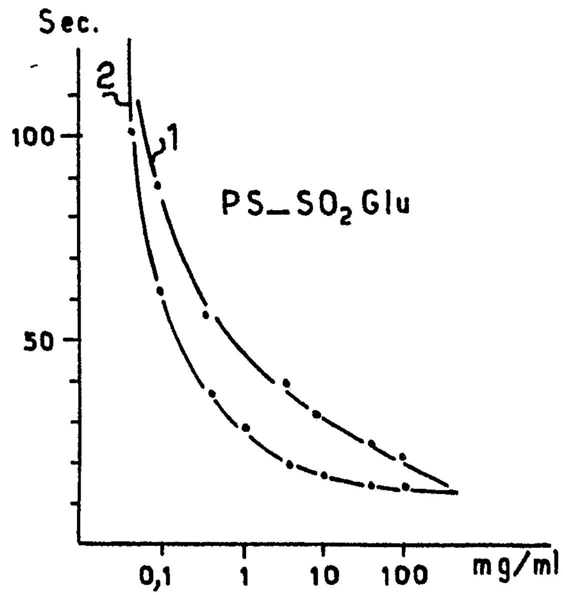


Fig.11b

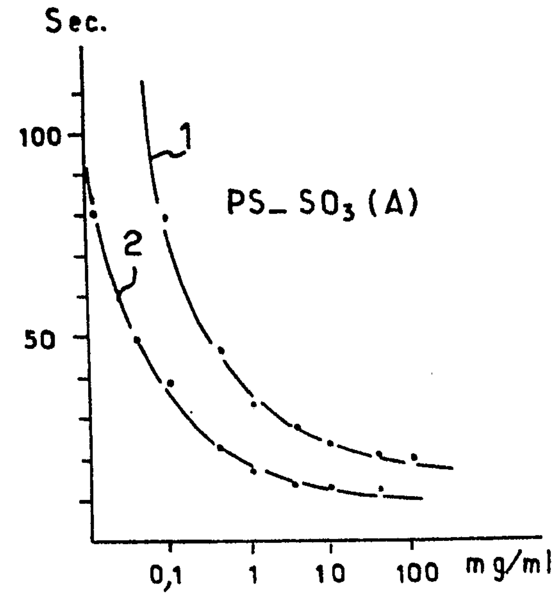

Fig. 4

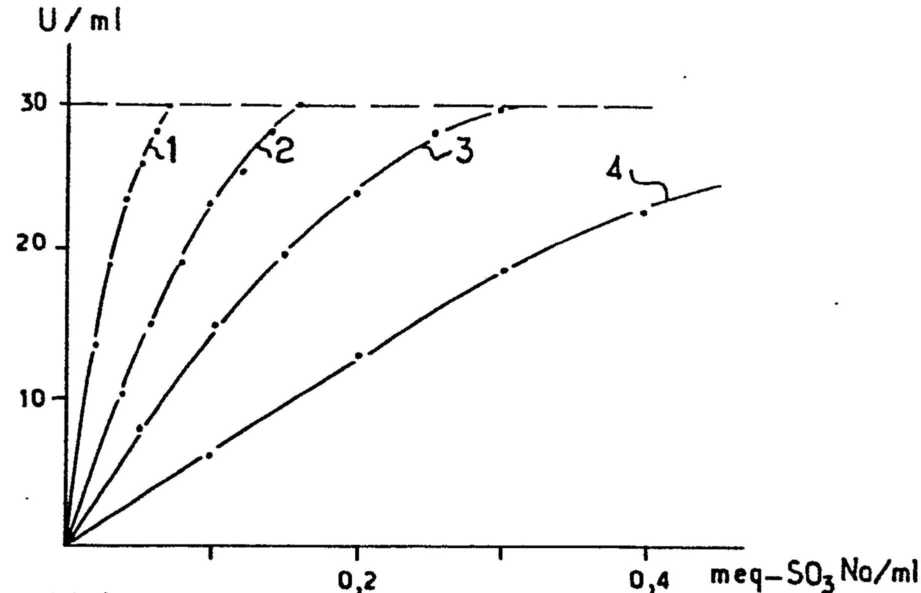


Fig. 5

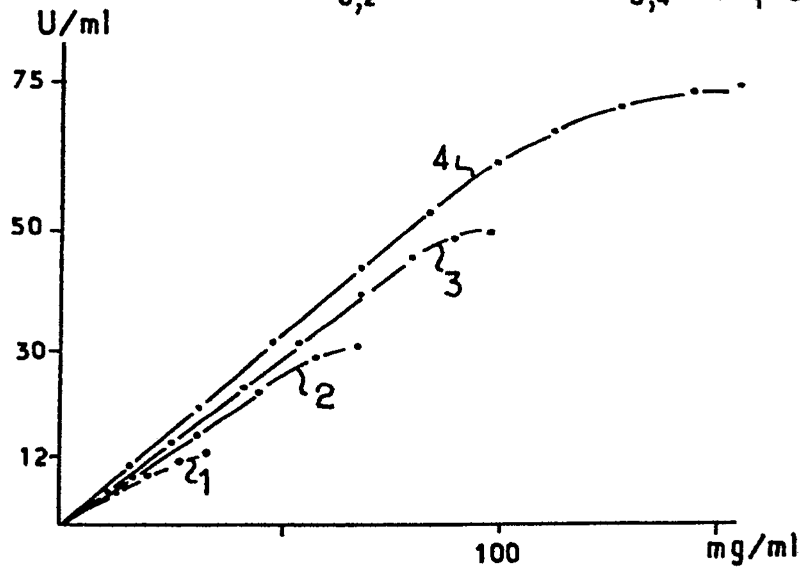


Fig. 6

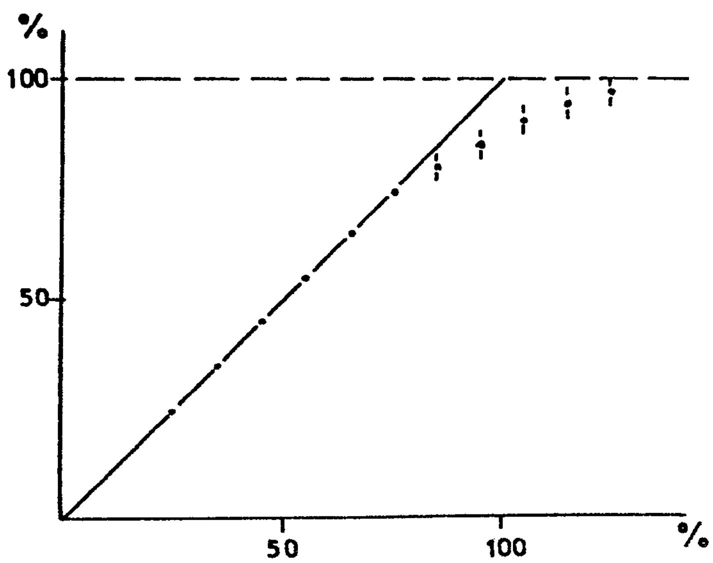

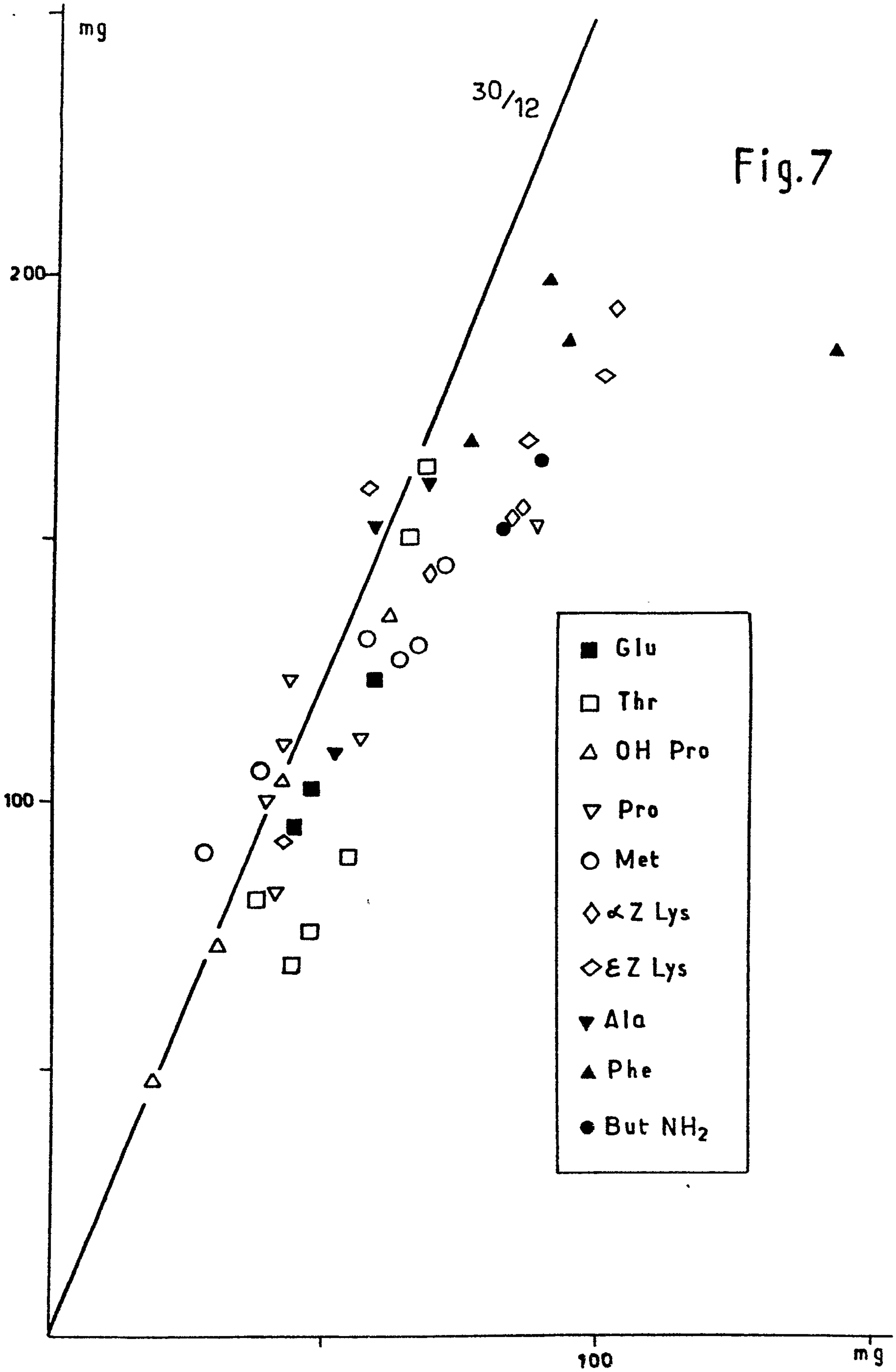
mg
200
100
30/12
100
mg
Glu
Thr
OH Pro
Pro
Met
αZ Lys
εZ Lys
Ala
Phe
But NH2

Fig.7

Fig. 8

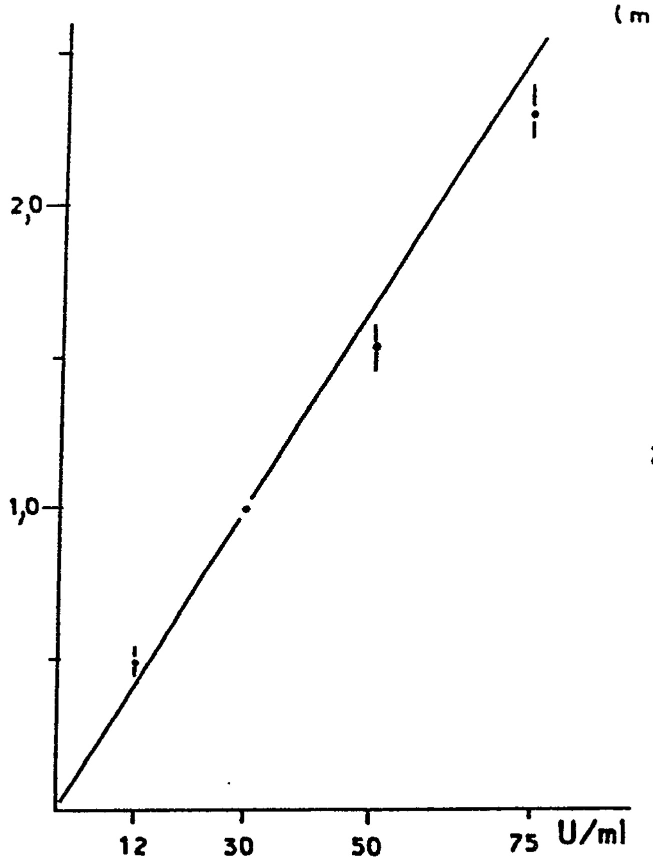


Fig. 10

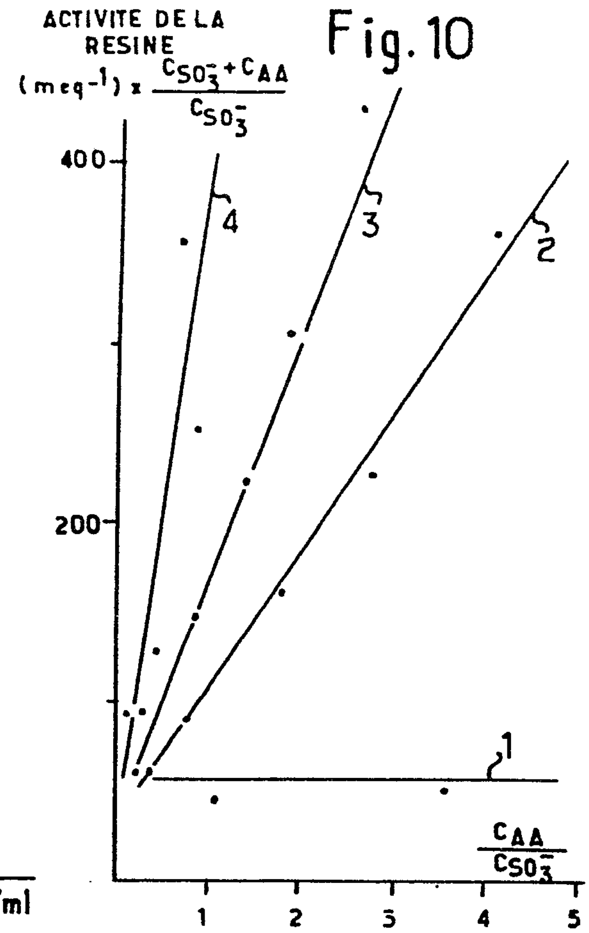


Fig. 9